# EUROPEAN PATENT SPECIFICATION

(11) **EP 2 335 654 B1**
(45) Date of publication and mention of the grant of the patent: **04.07.2018**
(21) Application number: 10012404.9
(22) Date of filing: 24.11.2004
(51) Int. Cl.: A61F 2/38, A61F 2/30

(54) **PATIENT SELECTABLE KNEE JOINT ARTHOPLASTY DEVICES**
PATIENTENABHÄNGIGE ARTHROPLASTIEVORRICHTUNG FÜR DAS KNIEGELENK
DISPOSITIFS D'ARTHROPLASTIE D'ARTICULATION DU GENOU SELECTIONNABLES SELON LE PATIENT

(30) Priority: 25.11.2003 US 724010; 05.01.2004 US 752438
(43) Date of publication of application: 22.06.2011
(62) Divisional of application: 04812273.3
(73) Proprietor: ConforMIS, Inc., Billerica, MA 01821 (US)
(72) Inventor: BURDULIS, Albert G. Jr., San Francisco, CA 94127 (US); FITZ, Wolfgang, Sherborn, MA 01770 (US); LANG, Philipp, Lexington, MA 02421 (US); STEINES, Daniel, Lexington, MA 02421 (US); TSOUGARAKIS, Konstantinos, Mountain View, CA 94041 (US); VARGAS-VORACEK, Rene, Sunnyvale, CA 94087 (US); BOUADI, Hacene, Palo Alto, CA 94306 (US); O'REGAN, Cecily Anne, East Falmouth, MA 02536 (US); MILLER, David, Cupertino, CA 95014 (US)
(74) Representative: Grund, Martin

(56) References cited:
- US-A- 3 798 679
- US-A- 4 822 365
- US-A- 5 728 162
- US-B1- 6 197 064

## Description

### FIELD OF THE INVENTION

The present invention relates to orthopedic devices and more particularly relates to devices for articular resurfacing in the knee. The closest prior art is document US 3798679 A, which defines the independent claims.

### BACKGROUND OF THE INVENTION

There are various types of cartilage, e.g., hyaline cartilage and fibrocartilage. Hyaline cartilage is found at the articular surfaces of bones, e.g., in the joints, and is responsible for providing the smooth gliding motion characteristic of moveable joints. Articular cartilage is firmly attached to the underlying bones and measures typically less than 5mm in thickness in human joints, with considerable variation depending on the joint and the site within the joint.

Adult cartilage has a limited ability of repair; thus, damage to cartilage produced by disease, such as rheumatoid and/or osteoarthritis, or trauma can lead to serious physical deformity and debilitation. Furthermore, as human articular cartilage ages, its tensile properties change. The superficial zone of the knee articular cartilage exhibits an increase in tensile strength up to the third decade of life, after which it decreases markedly with age as detectable damage to type II collagen occurs at the articular surface. The deep zone cartilage also exhibits a progressive decrease in tensile strength with increasing age, although collagen content does not appear to decrease. These observations indicate that there are changes in mechanical and, hence, structural organization of cartilage with aging that, if sufficiently developed, can predispose cartilage to traumatic damage.

Once damage occurs, joint repair can be addressed through a number of approaches. One approach includes the use of matrices, tissue scaffolds or other carriers implanted with cells (e.g., chondrocytes, chondrocyte progenitors, stromal cells, mesenchymal stem cells, etc.). These solutions have been described as a potential treatment for cartilage and meniscal repair or replacement. See, also, International Publications WO 99/51719 to Fofonoff, published October 14, 1999; WO01/91672 to Simon et al., published 12/6/2001; and WO01/17463 to Mannsmann, published March 15, 2001; U.S. Patent No. 6,283,980 B1 to Vibe-Hansen et al., issued September 4, 2001, U.S. Patent No. 5,842,477 to Naughton issued December 1, 1998, U.S. Patent No. 5,769,899 to Schwartz et al. issued June 23, 1998, U.S. Patent No. 4,609,551 to Caplan et al. issued September 2, 1986, U.S. Patent No. 5,041,138 to Vacanti et al. issued August 29, 1991, U.S. Patent No. 5,197,985 to Caplan et al. issued March 30, 1993, U.S. Patent No. 5,226,914 to Caplan et al. issued July 13, 1993, U.S. Patent No. 6,328,765 to Hardwick et al. issued December 11, 2001, U.S. Patent No. 6,281,195 to Rueger et al. issued August 28, 2001, and U.S. Patent No. 4,846,835 to Grande issued July 11, 1989. However, clinical outcomes with biologic replacement materials such as allograft and autograft systems and tissue scaffolds have been uncertain since most of these materials do not achieve a morphologic arrangement or structure similar to or identical to that of normal, disease-free human tissue it is intended to replace. Moreover, the mechanical durability of these biologic replacement materials remains uncertain.

Usually, severe damage or loss of cartilage is treated by replacement of the joint with a prosthetic material, for example, silicone, e.g. for cosmetic repairs, or metal alloys. See, e.g., U.S. Patent No. 6,383,228 to Schmotzer, issued May 7, 2002; U.S. Patent No. 6,203,576 to Afriat et al., issued March 20, 2001; U.S. Patent No. 6,126,690 to Ateshian, et al., issued October 3, 2000. Implantation of these prosthetic devices is usually associated with loss of underlying tissue and bone without recovery of the full function allowed by the original cartilage and, with some devices, serious long-term complications associated with the loss of significant amount of tissue and bone can include infection, osteolysis and also loosening of the implant.

Further, joint arthroplasties are highly invasive and require surgical resection of the entire articular surface of one or more bones, or a majority thereof. With these procedures, the marrow space is often reamed to fit the stem of the prosthesis. The reaming results in a loss of the patient's bone stock. U.S. Patent 5,593,450 to Scott et al. issued January 14, 1997 discloses an oval domed shaped patella prosthesis. The prosthesis has a femoral component that includes two condyles as articulating surfaces. The two condyles meet to form a second trochlear groove and ride on a tibial component that articulates with respect to the femoral component. A patella component is provided to engage the trochlear groove. U.S. Patent 6,090,144 to Letot et al. issued July 18, 2000 discloses a knee prosthesis that includes a tibial component and a meniscal component that is adapted to be engaged with the tibial component through an asymmetrical engagement.

A variety of materials can be used in replacing a joint with a prosthetic, for example, silicone, e.g. for cosmetic repairs, or suitable metal alloys are appropriate. See, e.g., U.S. Patent No. 6,443,991 B1 to Running issued September 3, 2002, U.S. Patent No. 6,387,131 B1 to Miehlke et al. issued May 14, 2002; U.S. Patent No. 6,383,228 to Schmotzer issued May 7, 2002; U.S. Patent No. 6,344,059 B1 to Krakovits et al. issued February 5, 2002; U.S. Patent No. 6,203,576 to Afriat et al. issued March 20, 2001; U.S. Patent No. 6,126,690 to Ateshian et al. issued October 3, 2000; U.S. Patent 6,013,103 to Kaufman et al. issued January 11, 2000. Implantation of these prosthetic devices is usually associated with loss of underlying tissue and bone without recovery of the full function allowed by the original cartilage and, with some devices, serious long-term complications associated with the loss of significant amounts of tissue and bone can cause loosening of the implant. One such complication is osteolysis. Once the prosthesis becomes loosened from the joint, regardless of the cause, the prosthesis will then need to be replaced. Since the patient's bone stock is limited, the number of possible replacement surgeries is also limited for joint arthroplasty.

As can be appreciated, joint arthroplasties are highly invasive and require surgical resection of the entire, or a majority of the, articular surface of one or more bones involved in the repair. Typically with these procedures, the marrow space is fairly extensively reamed in order to fit the stem of the prosthesis within the bone. Reaming results in a loss of the patient's bone stock and over time subsequent osteolysis will frequently lead to loosening of the prosthesis. Further, the area where the implant and the bone mate degrades over time requiring the prosthesis to eventually be replaced. Since the patient's bone stock is limited, the number of possible replacement surgeries is also limited for joint arthroplasty. In short, over the course of 15 to 20 years, and in some cases even shorter time periods, the patient can run out of therapeutic options ultimately resulting in a painful, non-functional joint.

U.S. Patent No. 6,206,927 to Fell, et al., issued March 27, 2001, and U.S. Patent No. 6,558,421 to Fell, et al., issued May 6, 2003, disclose a surgically implantable knee prosthesis that does not require bone resection. This prosthesis is described as substantially elliptical in shape with one or more straight edges. Accordingly, these devices are not designed to substantially conform to the actual shape (contour) of the remaining cartilage in vivo and/or the underlying bone. Thus, integration of the implant can be extremely difficult due to differences in thickness and curvature between the patient's surrounding cartilage and/or the underlying subchondral bone and the prosthesis. U.S. Patent 6,554,866 to Aicher, et al. issued April 29, 2003 describes a mono-condylar knee joint prosthesis.

Interpositional knee devices that are not attached to both the tibia and femur have been described. For example, Platt et al. (1969) "Mould Arthroplasty of the Knee," Journal of Bone and Joint Surgery 51B(1):76-87, describes a hemi-arthroplasty with a convex undersurface that was not rigidly attached to the tibia. Devices that are attached to the bone have also been described. Two attachment designs are commonly used. The McKeever design is a cross-bar member, shaped like a "t" from a top perspective view, that extends from the bone mating surface of the device such that the "t" portion penetrates the bone surface while the surrounding surface from which the "t" extends abuts the bone surface. See McKeever, "Tibial Plateau Prosthesis," Chapter 7, p. 86. An alternative attachment design is the Macintosh design, which replaces the "t" shaped fin for a series of multiple flat serrations or teeth. *See* Potter, "Arthroplasty of the Knee with Tibial Metallic Implants of the McKeever and Macintosh Design," Surg. Clins. Of North Am. 49(4): 903-915 (1969).

U.S. Patent 4,502,161 to Wall issued March 5, 1985, describes a prosthetic meniscus constructed from materials such as silicone rubber or Teflon with reinforcing materials of stainless steel or nylon strands. U.S. Patent 4,085,466 to Goodfellow et al. issued March 25, 1978, describes a meniscal component made from plastic materials. Reconstruction of meniscal lesions has also been attempted with carbon-fiber-polyurethane-poly (L-lactide). Leeslag, et al., Biological and Biomechanical Performance of Biomaterials (Christel et al., eds.) Elsevier Science Publishers B.V., Amsterdam. 1986. pp. 347-352. Reconstruction of meniscal lesions is also possible with bioresorbable materials and tissue scaffolds.

However, currently available devices do not always provide ideal alignment with the articular surfaces and the resultant joint congruity. Poor alignment and poor joint congruity can, for example, lead to instability of the joint. Further, none of these solutions take into account the fact that roughly 80% of patients undergoing knee surgery have a healthy lateral compartment and only need to repair the medial condyle and the patella. An additional 10% only have damage to the lateral condyle. Thus, 90% of patients do not require the entire condylar surface repaired.

Thus, there remains a need for compositions for joint repair, including methods and compositions that facilitate the integration between the cartilage replacement system and the surrounding cartilage which takes into account the actual damage to be repaired. Further, there is a need for an implant or implant system that improves the anatomic result of the joint correction procedure by providing surfaces that more closely resemble the natural knee joint anatomy of a patient. Additionally, what is needed is an implant or implant system that provides an improved functional joint.

### SUMMARY OF THE INVENTION

The present invention is defined in independent claims 1 and 9 and provides novel devices for replacing a portion (*e.g*., diseased area and/or area slightly larger than the diseased area) of a knee joint (*e.g*., cartilage, meniscus and/or bone) with one or more implants, where the implant(s) achieves an anatomic or near anatomic fit with the surrounding structures and tissues. In cases where the devices include an element associated with the underlying articular bone, the invention also provides that the bone-associated element can achieve a near anatomic alignment with the subchondral bone. The invention may be used in a method providing the preparation of an implantation site with a single cut, or a few relatively small cuts. Asymmetrical components can also be provided to improve the anatomic functionality of the repaired joint by providing a solution that closely resembles the natural knee joint anatomy. The improved anatomic results, in turn, leads to an improved functional result for the repaired joint. The invention also provides a kit which includes one or more implants used to achieve optimal joint correction.

### BRIEF DESCRIPTION OF THE DRAWINGS

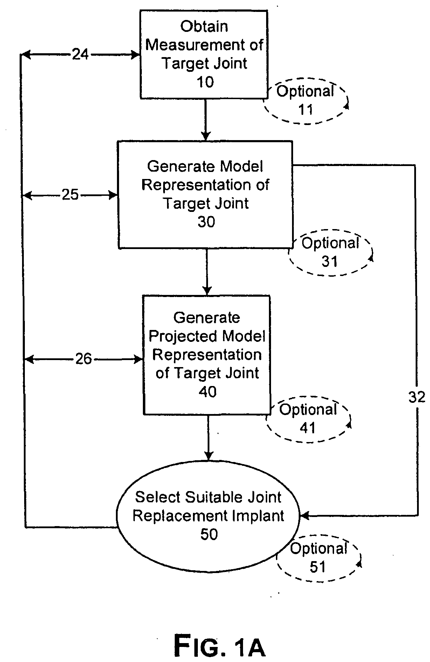

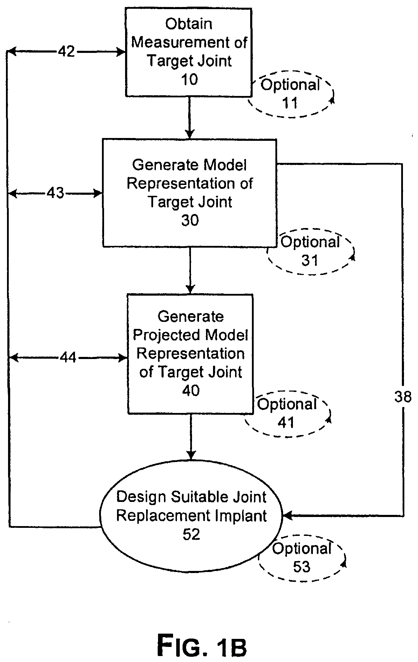

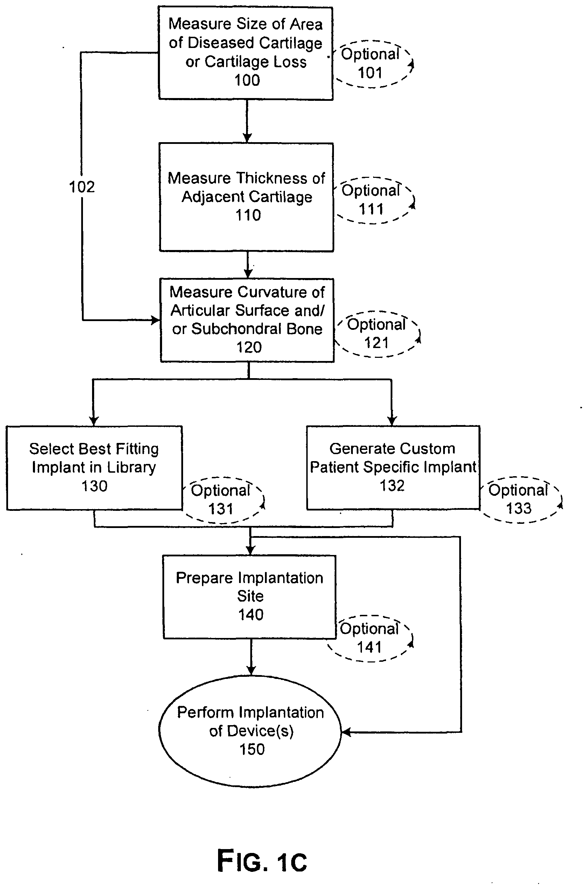

**FIG. 1A** is a block diagram of a method for assessing a joint in need of repair according to the invention wherein the existing joint surface is unaltered, or substantially unaltered, prior to receiving the selected implant. **FIG. 1B** is a block diagram of a method for assessing a joint in need of repair according to the invention wherein the existing joint surface is unaltered, or substantially unaltered, prior to designing an implant suitable to achieve the repair. **FIG. 1C** is a block diagram of a method for developing an implant and using the implant in a patient.

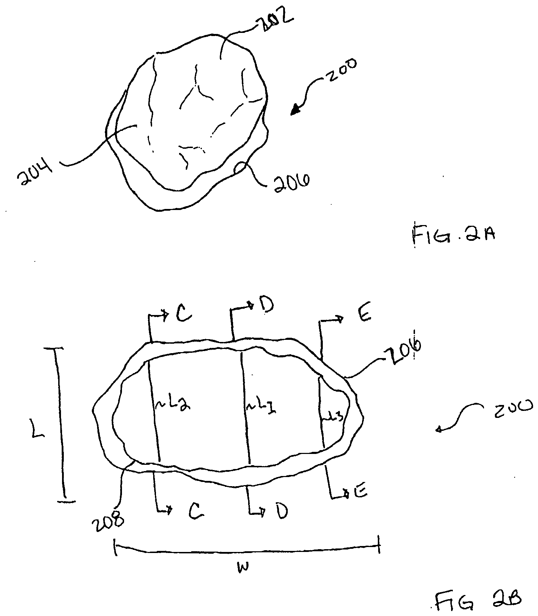

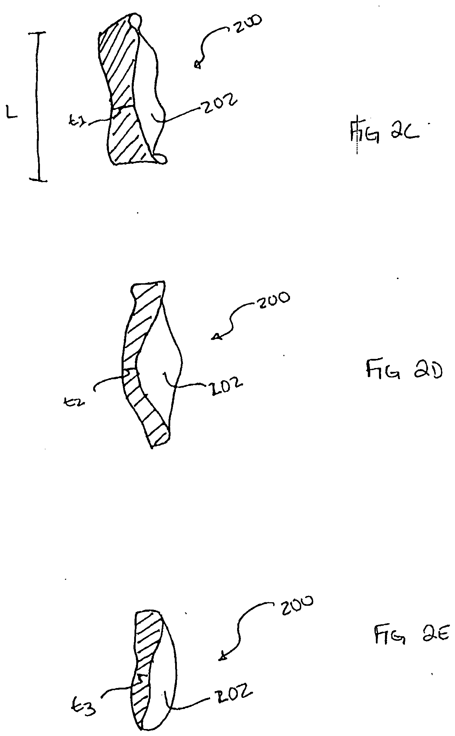

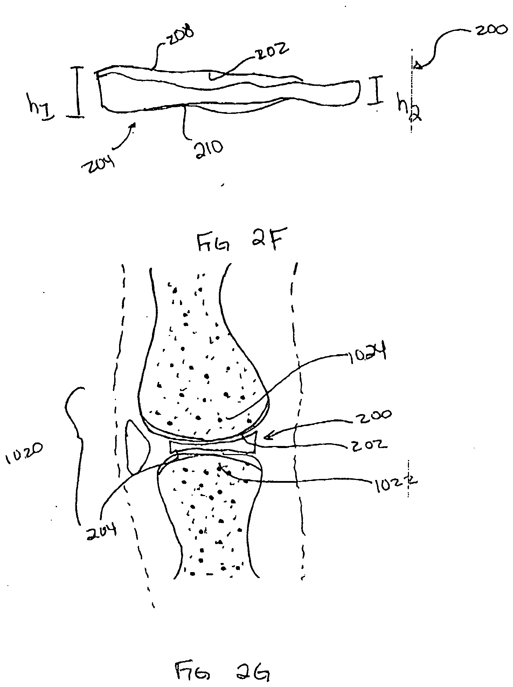

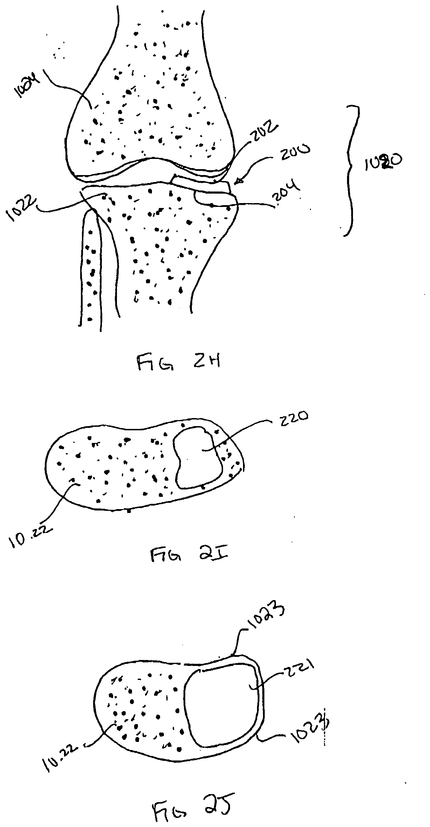

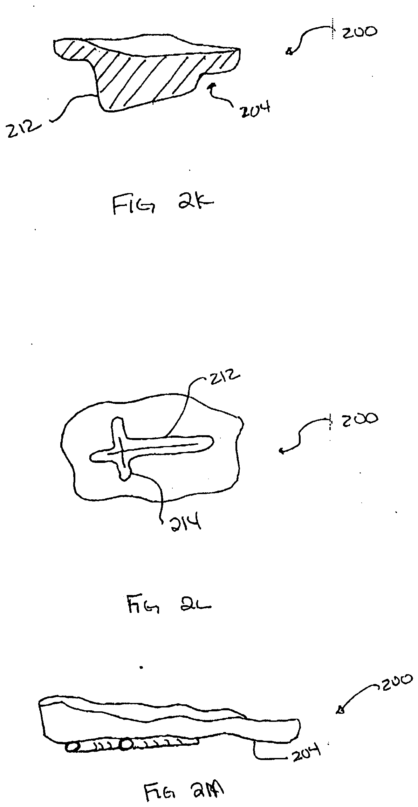

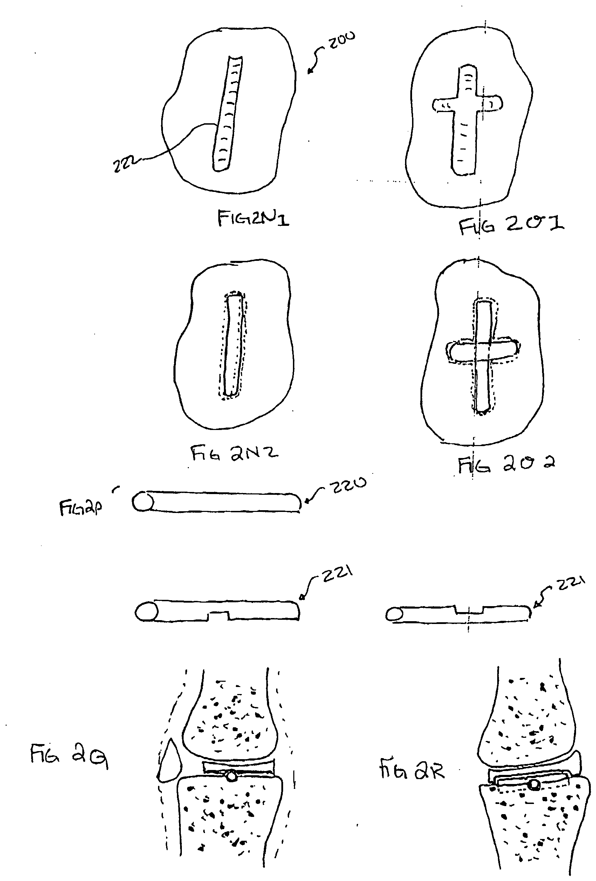

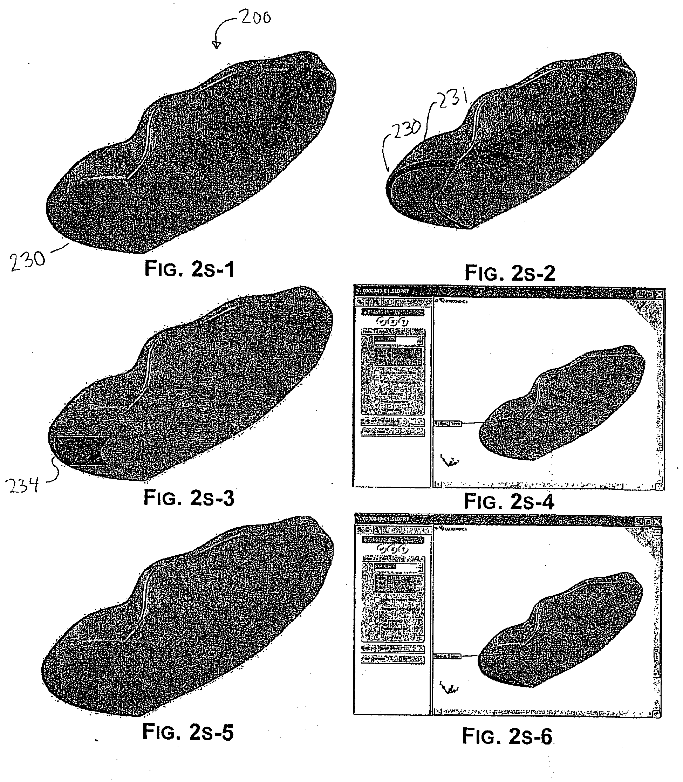

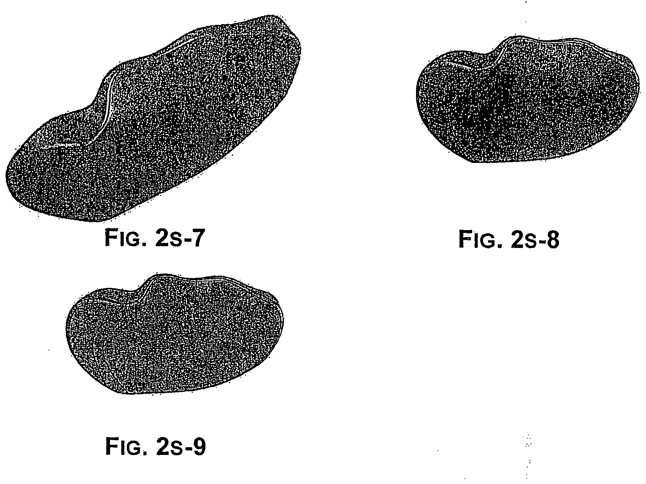

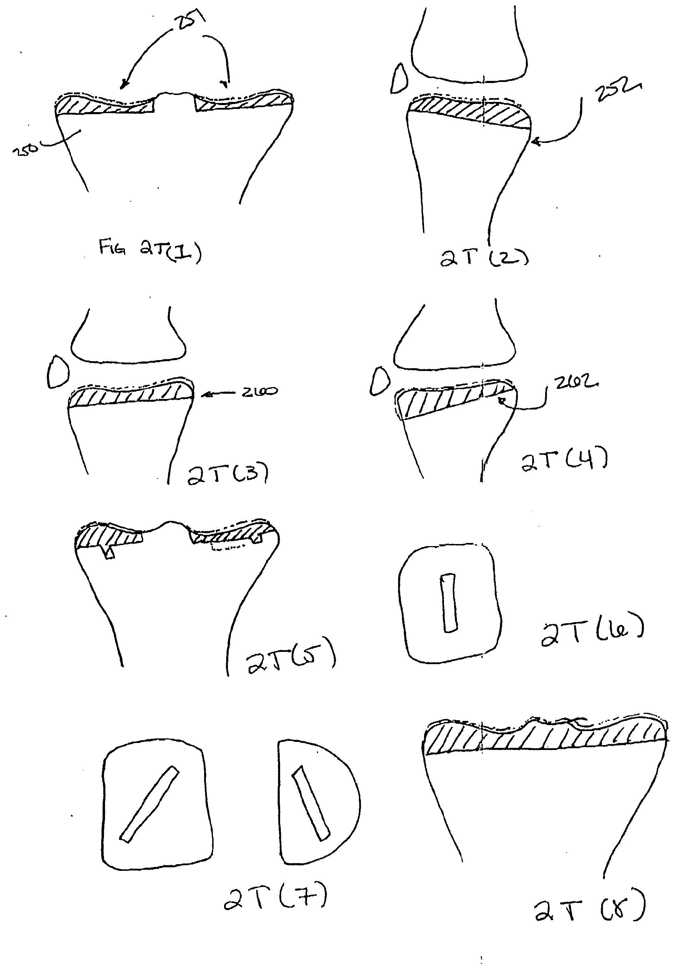

**FIG. 2A** is a perspective view of a joint implant suitable for implantation at the tibial plateau of the knee joint. **FIG. 2B** is a top view of the implant of **FIG. 2A****.** **FIG. 2C** is a cross-sectional view of the implant of **FIG. 2B** along the lines C-C shown in **FIG. 2B****.** **FIG. 2D** is a cross-sectional view along the lines D-D shown in **FIG. 2B****.** **FIG. 2E** is a cross-sectional view along the lines E-E shown in **FIG. 2B****.** **FIG. 2F** is a side view of the implant of **FIG. 2A****.** **FIG. 2G** is a cross-sectional view of the implant of **FIG. 2A** shown implanted taken along a plane parallel to the sagittal plane. **FIG. 2H** is a cross-sectional view of the implant of **FIG. 2A** shown implanted taken along a plane parallel to the coronal plane. **FIG. 2I** is a cross-sectional view of the implant of **FIG. 2A** shown implanted taken along a plane parallel to the axial plane. **FIG. 2J** shows a slightly larger implant that extends closer to the bone medially (towards the edge of the tibial plateau) and anteriorly and posteriorly. **FIG. 2K** is a side view of an alternate embodiment of the joint implant of **FIG. 2A** showing an anchor in the form of a keel. **FIG. 2L** is a bottom view of an alternate embodiment of the joint implant of **FIG. 2A** showing an anchor. **FIG. 2M** shows an anchor in the form of a cross-member. **FIG. 2N-O** are alternative embodiments of the implant showing the lower surface have a trough for receiving a cross-bar. **FIG. 2P** illustrates a variety of cross-bars. **FIGS. 2Q-R** illustrate the device implanted within a knee joint. **FIGS. 2S(1-9)** illustrate another implant suitable for the tibial plateau further having a chamfer cut along one edge. **FIG. 2T(1-8)** illustrate an alternate embodiment of the tibial implant wherein the surface of the joint is altered to create a flat or angled surface for the implant to mate with.

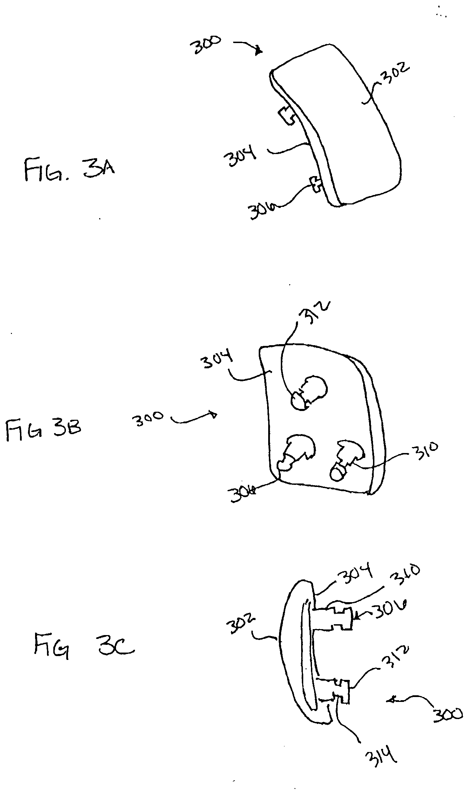

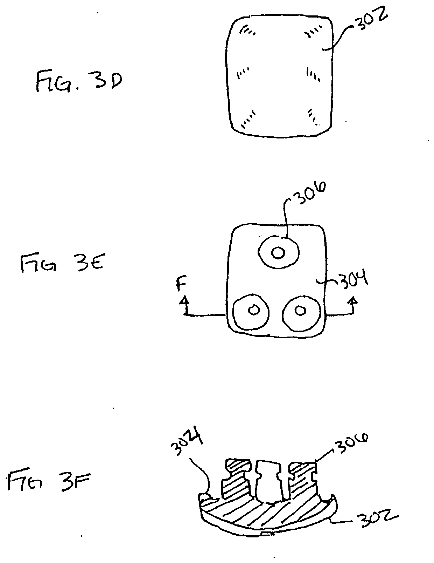

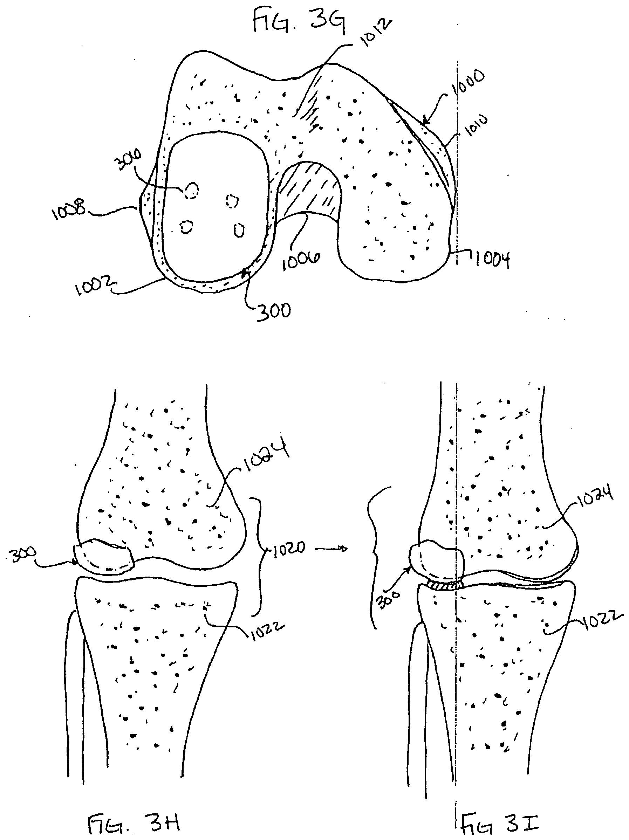

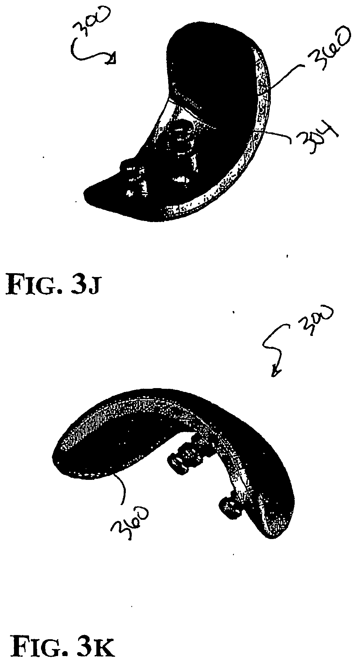

**FIGS. 3A** and **B** are perspective views of a joint implant suitable for use on a condyle of the femur from the inferior and superior surface viewpoints, respectively. **FIG. 3c** is a side view of the implant of **FIG. 3A****.** **FIG. 3D** is a view of the inferior surface of the implant; **FIG. 3E** is a view of the superior surface of the implant and **FIG. 3F** is a cross-section of the implant. **FIG. 3G** is an axial view of a femur with the implant installed thereon. **FIG. 3H** is an anterior view of the knee joint without the patella wherein the implant is installed on the femoral condyle. **FIG. 3I** is an anterior view of the knee joint with an implant of **FIG. 3A** implanted on the femoral condyle along with an implant suitable for the tibial plateau, such as that shown in **FIG. 2****.** **FIGS. 3J-K** illustrate an alternate example of a joint implant for use on a condyle of a femur further having at least one chamfer cut.

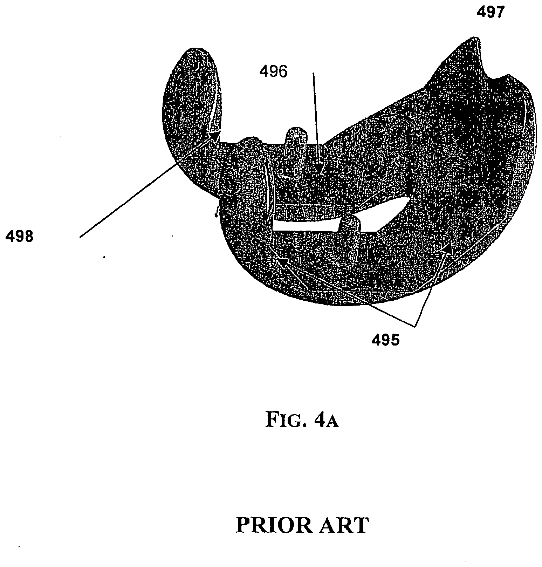

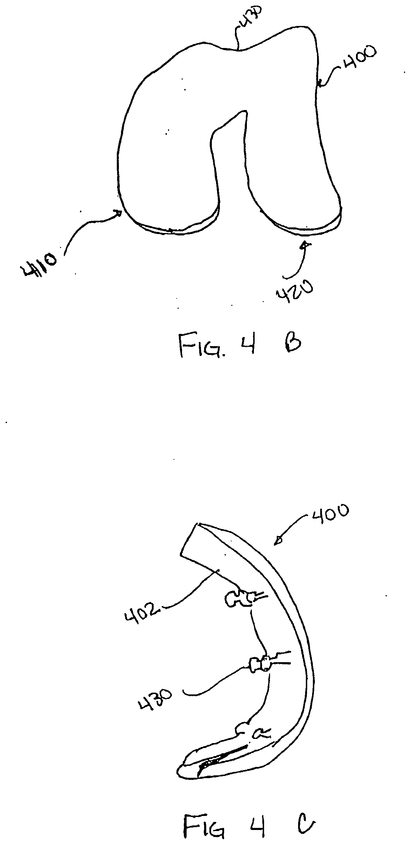

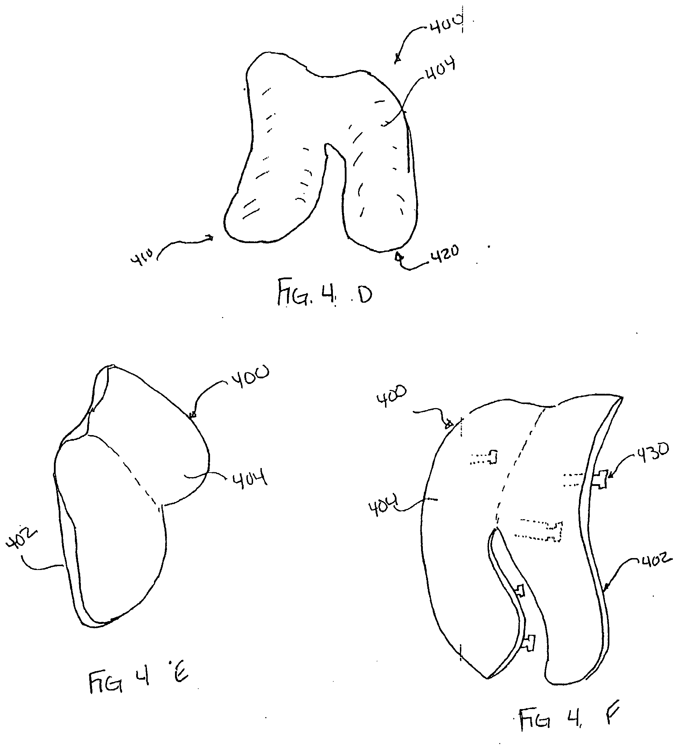

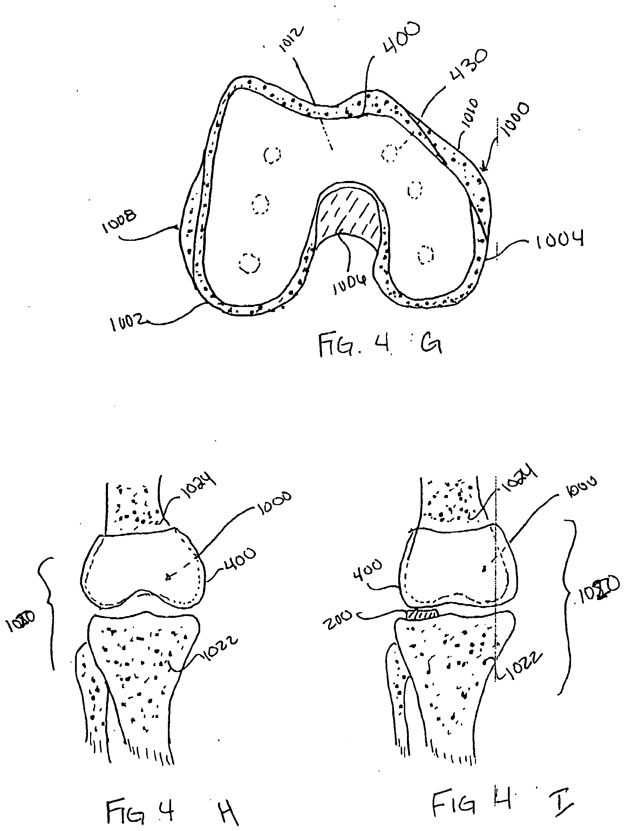

**FIG. 4A** illustrates an implant suitable for the femoral condyle according to the prior art. **FIGS. 48-I** depict another implant suitable for placement on a femoral condyle. **FIG. 4B** is a slightly perspective view of the implant from the superior surface. **FIG. 4C** is a side view of the implant of **FIG. 4B****.** **FIG. 4D** is a top view of the inferior surface of the implant; **FIG. 4E** and **F** are perspective side views of the implant. **FIG. 4G** is an axial view of a femur with the implant installed thereon. **FIG. 4H** is an anterior view of the knee joint without the patella wherein the implant is installed on the femoral condyle. **FIG. 4I** is an anterior view of the knee joint with an implant of **FIG. 4B** implanted on the femoral condyle along with an implant suitable for the tibial plateau, such as that shown in **FIG. 2****.**

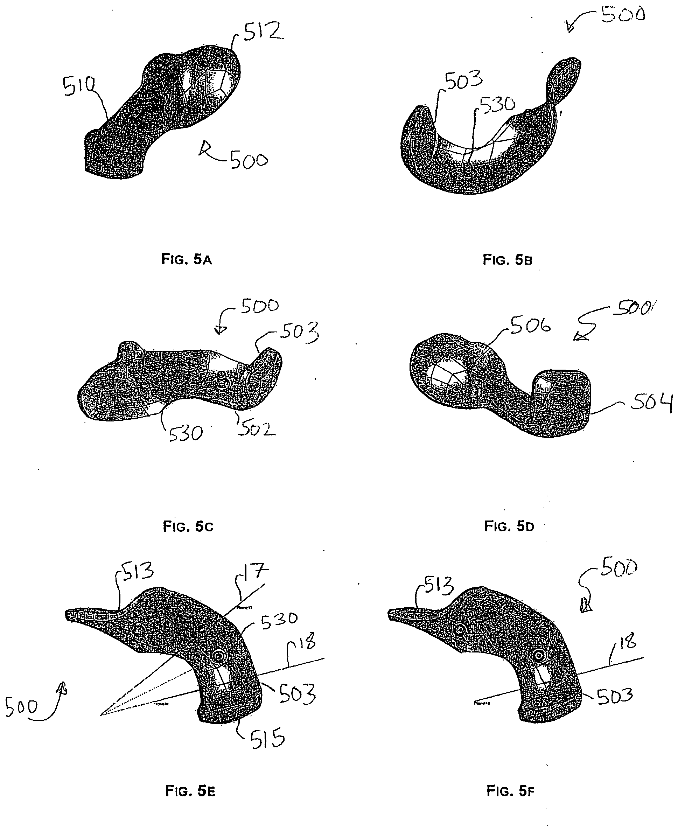

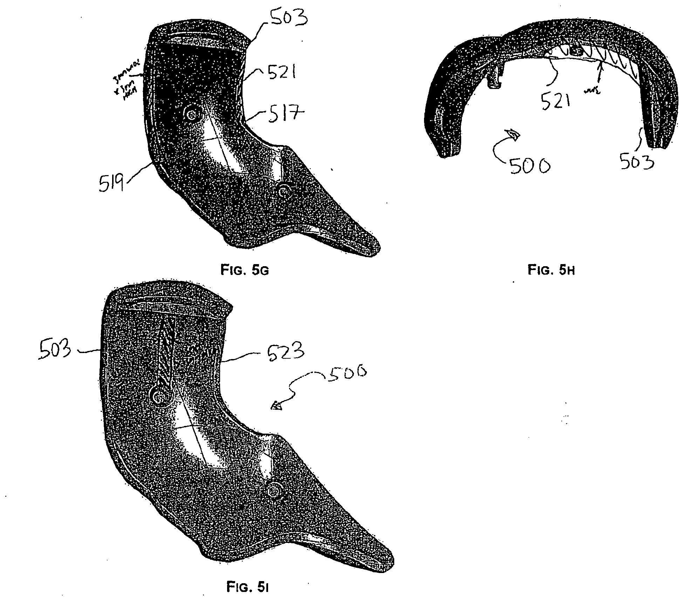

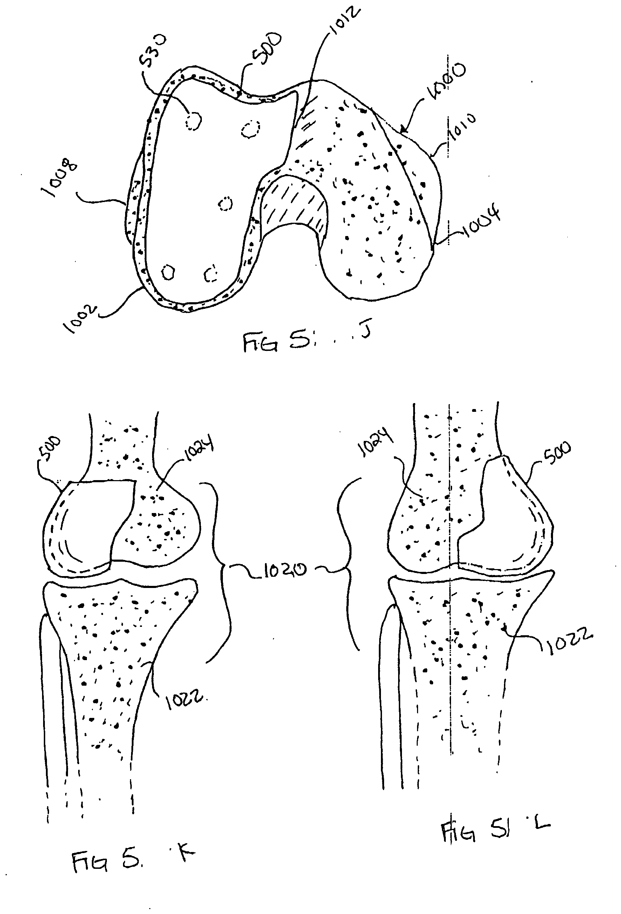

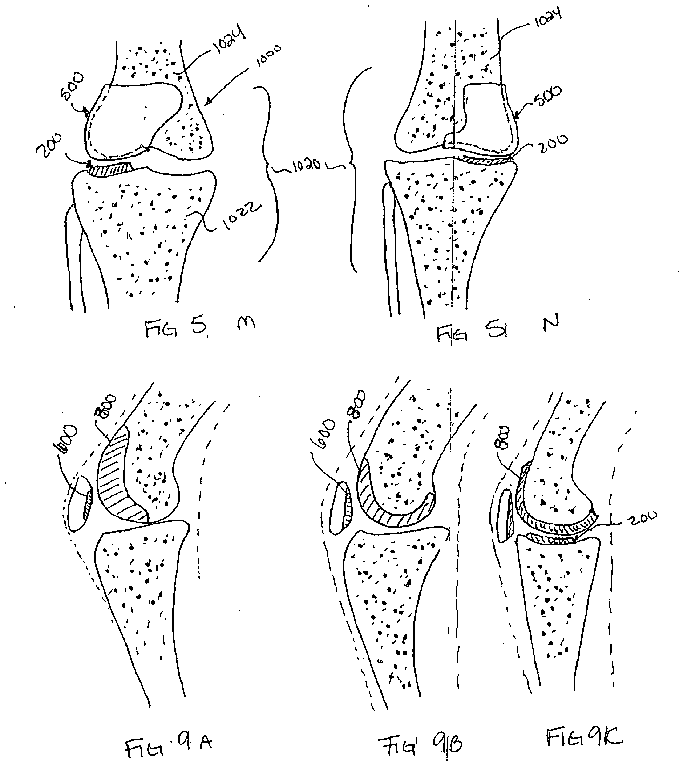

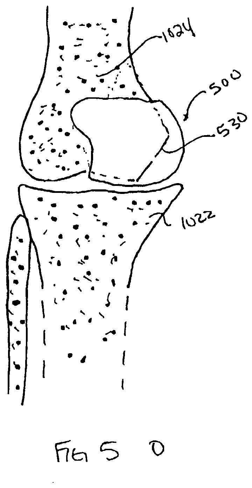

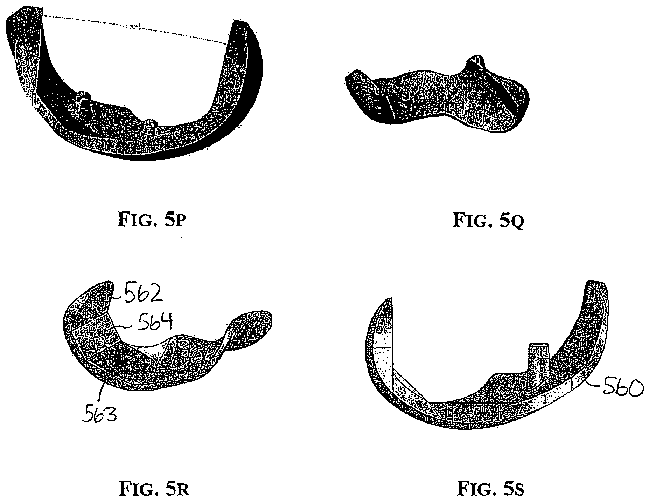

**FIGS. 5A-S** are depictions of another implant according to the invention suitable for placement on the femoral condyle. **FIG. 5A** is a top view of the inferior surface of the implant showing a chamfer cut. **FIG. 5B** is a slightly perspective view of the superior surface of the implant. **FIG. 5C** is a perspective side view of the implant from a first direction; **FIG. 5D** is a slightly perspective side view of the implant from a second direction. **FIGS. 5E-F** are side views of the implant showing the bearing loads; **FIGS. 5G** and **H** illustrate an alternative embodiment wherein the implant has lateral rails; **FIG. 5I** illustrates another embodiment wherein the implant has an anchoring keel. **FIG. 5J** is an axial view of a femur with the implant installed on the femoral condyles. **FIG. 5K** is an anterior view of the knee joint without the patella wherein the implant is installed on the femoral condyle. **FIG. 5L** is an anterior view of the knee joint with an implant of **FIG. 5A** implanted on the femoral condyles along with an implant suitable for the tibial plateau, such as that shown in **FIG. 2****.** **FIGS. 5M-N** depicts a device implanted within the knee joint. **FIG. 5O** depicts an alternate embodiment of the device which accommodates an partial removal of the condyle. **FIGS. 5P-S** illustrate alternative embodiments of the implant having one or more chamfer cuts.

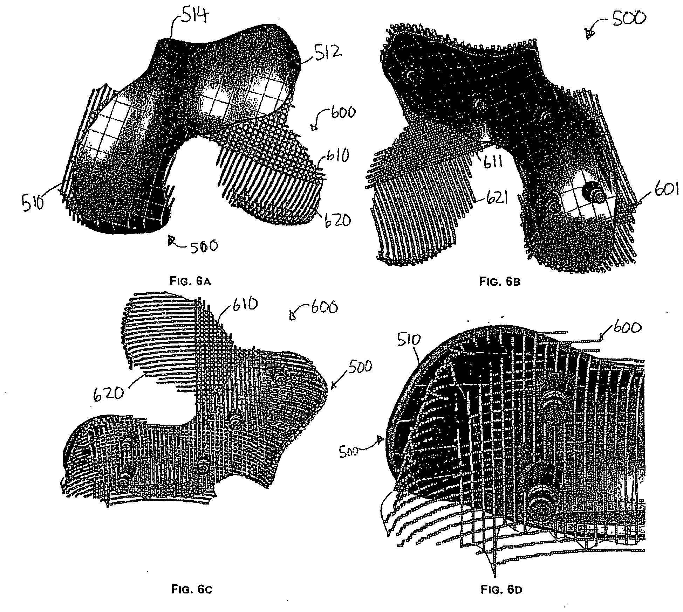

**FIGS. 6A-G** illustrate a device as shown in **FIG. 5** along with a graphical representation of the cross-sectional data points comprising the surface map.

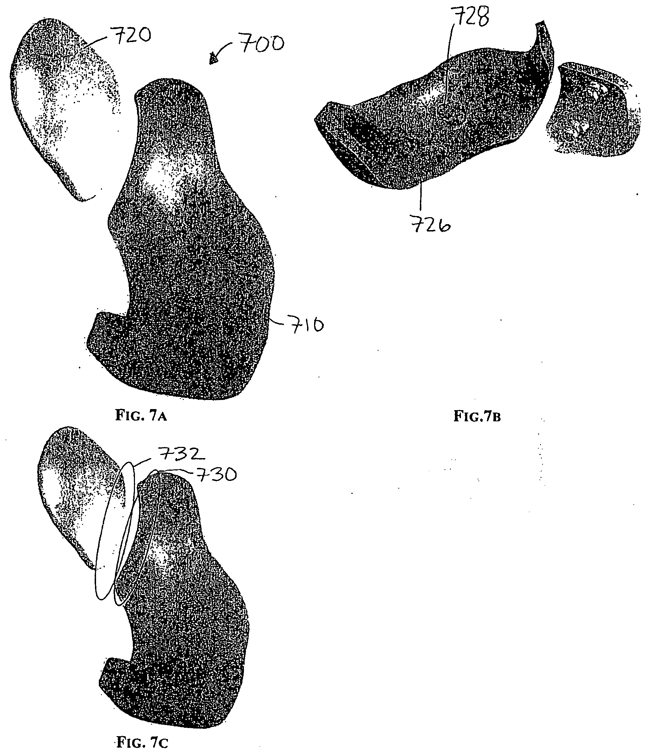

**FIGS. 7A-C** illustrate an alternate design of a device, suitable for a portion of the femoral condyle, having a two piece configuration.

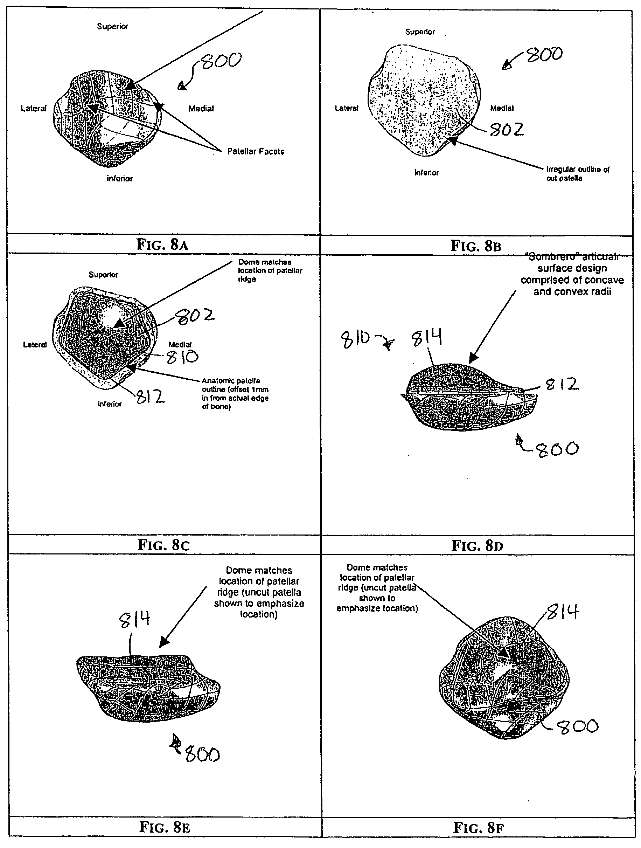

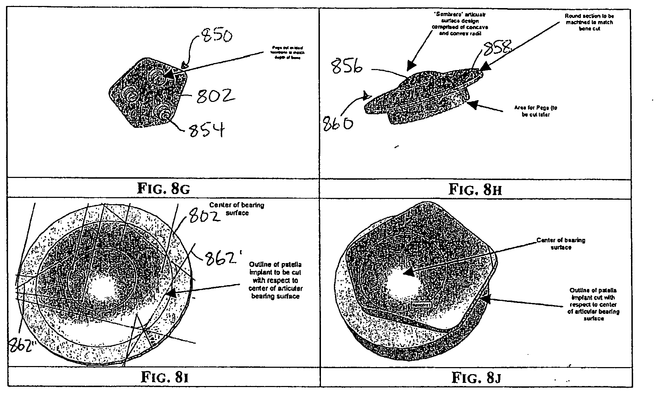

**FIGS. 8A-J** depict a whole patella **(****FIG. 8A****)** and a patella that has been cut in order to install an implant **(****FIG. 8B****).** A top and side view of a suitable patella implant is shown **(****FIGS. 8C-D),** and an illustration of the implant superimposed on a whole patella is shown to illustrate the location of the implant dome relative to the patellar ridge. FIGS. **8E-F** illustrate the implant superimposed over a patella. **FIGS. 8G-J** illustrate an alternate design for the patella implant basedon a blank **(****FIG. 8G****).**
FIGS. **9A-C** depict representative side views of a knee joint with any of the devices taught installed therein. **FIG. 9A** depicts the knee with a condyle implant and a patella implant. **FIG. 9B** depicts an alternate view of the knee with a condyle implant and a patella implant wherein the condyle implant covers a greater portion of the surface of the condyle in the posterior direction. **FIG. 9c** illustrates a knee joint wherein the implant is provided on the condyle, the patella and the tibial plateau.

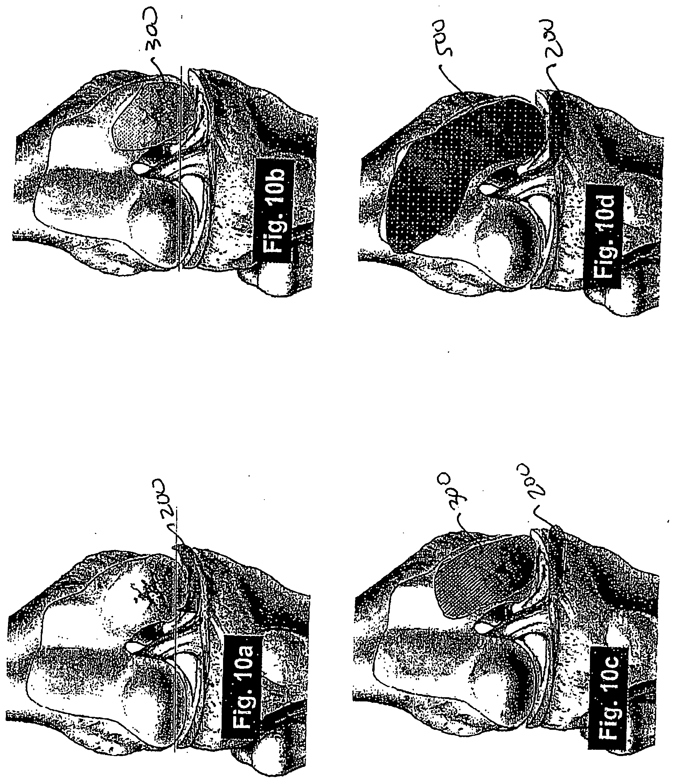

**FIGS. 10A-D** depict a frontal view of the knee joint with any of the devices taught installed therein. **FIG. 10A** depicts the knee with a tibial implant. **FIG. 10B** depicts the knee with a condyle implant. **FIG. 10c** depicts a knee with a tibial implant and a condyle implant. **FIG. 10c** depicts a knee with a bicompartmental condyle implant and a tibial implant.

### DETAILED DESCRIPTION OF THE INVENTION

The following description is presented to enable any person skilled in the art to make and use the invention. Various modifications to the embodiments described will be readily apparent to those skilled in the art, and the generic principles defined herein can be applied to other embodiments and applications without departing from the scope of the present invention as defined by the appended claims. Thus, the present invention is not intended to be limited to the embodiments shown, but is to be accorded the widest scope consistent with the principles and features disclosed herein.

As will be appreciated by those of skill in the art, methods recited herein may be carried out in any order of the recited events which is logically possible, as well as the recited order of events. Furthermore, where a range of values is provided, it is understood that every intervening value, between the upper and lower limit of that range and any other stated or intervening value in that stated range is encompassed within the invention. Also, it is contemplated that any optional feature of the inventive variations described may be set forth and claimed independently, or in combination with any one or more of the features described herein.

The practice of the present invention can employ, unless otherwise indicated, conventional and digital methods of x-ray imaging and processing, x-ray tomosynthesis, ultrasound including A-scan, B-scan and C-scan, computed tomography (CT scan), magnetic resonance imaging (MRI), optical coherence tomography, single photon emission tomography (SPECT) and positron emission tomography (PET) within the skill of the art. Such techniques are explained fully in the literature and need not be described herein. See, *e.g*., X-Ray Structure Determination: A Practical Guide, 2nd Edition, editors Stout and Jensen, 1989, John Wiley & Sons, publisher; Body CT: A Practical Approach, editor Slone, 1999, McGraw-Hill publisher; X-ray Diagnosis: A Physician's Approach, editor Lam, 1998 Springer-Verlag, publisher; and Dental Radiology: Understanding the X-Ray Image, editor Laetitia Brocklebank 1997, Oxford University Press publisher. See *also,* The Essential Physics of Medical Imaging (2nd Ed.), Jerrold T. Bushberg, et al.

The present invention relates to methods and compositions for repairing joints, particularly for repairing articular cartilage and for facilitating the integration of a wide variety of cartilage repair materials into a subject. Among other things, the techniques described herein allow for the customization of cartilage repair material to suit a particular subject, for example in terms of size, cartilage thickness and/or curvature. When the shape (*e.g*., size, thickness and/or curvature) of the articular cartilage surface is an exact or near anatomic fit with the non-damaged cartilage or with the subject's original cartilage, the success of repair is enhanced. The repair material can be shaped prior to implantation and such shaping can be based, for example, on electronic images that provide information regarding curvature or thickness of any "normal" cartilage surrounding the defect and/or on curvature of the bone underlying the defect. Thus, the current invention provides, among other things, for minimally invasive methods for partial joint replacement. The methods will require only minimal or, in some instances, no loss in bone stock. Additionally, unlike with current techniques, the methods described herein will help to restore the integrity of the articular surface by achieving an exact or near anatomic match between the implant and the surrounding or adjacent cartilage and/or subchondral bone.

Advantages of the present invention can include, but are not limited to, (i) customization of joint repair, thereby enhancing the efficacy and comfort level for the patient following the repair procedure; (ii) eliminating the need for a surgeon to measure the defect to be repaired intraoperatively in some embodiments; (iii) eliminating the need for a surgeon to shape the material during the implantation procedure; (iv) providing methods of evaluating curvature of the repair material based on bone or tissue images or based on intraoperative probing techniques; (v) providing methods of repairing joints with only minimal or, in some instances, no loss in bone stock; (vi) improving postoperative joint congruity; (vii) improving the postoperative patient recovery in some embodiments and (viii) improving postoperative function, such as range of motion.

Thus, the methods described herein allow for the design and use of joint repair material that more precisely fits the defect (e.g., site of implantation) or the articular surface(s) and, accordingly, provides improved repair of the joint.

### I. ASSESSMENT OF JOINTS AND ALIGNMENT

The methods and compositions described herein can be used to treat defects resulting from disease of the cartilage (e.g., osteoarthritis), bone damage, cartilage damage, trauma, and/or degeneration due to overuse or age. The invention allows, among other things, a health practitioner to evaluate and treat such defects. The size, volume and shape of the area of interest can include only the region of cartilage that has the defect, but preferably will also include contiguous parts of the cartilage surrounding the cartilage defect.

As will be appreciated by those of skill in the art, size, curvature and/or thickness measurements can be obtained using any suitable technique. For example, one-dimensional, two-dimensional, and/or three-dimensional measurements can be obtained using suitable mechanical means, laser devices, electromagnetic or optical tracking systems, molds, materials applied to the articular surface that harden and "memorize the surface contour," and/or one or more imaging techniques known in the art. Measurements can be obtained non-invasively and/or intraoperatively (e.g., using a probe or other surgical device). As will be appreciated by those of skill in the art, the thickness of the repair device can vary at any given point depending upon patient's anatomy and/or the depth of the damage to the cartilage and/or bone to be corrected at any particular location on an articular surface.

**FIG. 1A** is a flow chart showing steps taken by a practitioner in assessing a joint. First, a practitioner obtains a measurement of a target joint ***10*.** The step of obtaining a measurement can be accomplished by taking an image of the joint. This step can be repeated, as necessary, ***11*** to obtain a plurality of images in order to further refine the joint assessment process. Once the practitioner has obtained the necessary measurements, the information is used to generate a model representation of the target joint being assessed ***30***. This model representation can be in the form of a topographical map or image. The model representation of the joint can be in one, two, or three dimensions. It can include a physical model. More than one model can be created **31,** if desired. Either the original model, or a subsequently created model, or both can be used. After the model representation of the joint is generated ***30,*** the practitioner can optionally generate a projected model representation of the target joint in a corrected condition ***40*,** e.g., from the existing cartilage on the joint surface, by providing a mirror of the opposing joint surface, or a combination thereof Again, this step can be repeated ***41*,** as necessary or desired. Using the difference between the topographical condition of the joint and the projected image of the joint, the practitioner can then select a joint implant ***50*** that is suitable to achieve the corrected joint anatomy. As will be appreciated by those of skill in the art, the selection process ***50*** can be repeated ***51*** as often as desired to achieve the desired result. Additionally, it is contemplated that a practitioner can obtain a measurement of a target joint ***10*** by obtaining, for example, an x-ray, and then select a suitable joint replacement implant ***50***.

As will be appreciated by those of skill in the art, the practitioner can proceed directly from the step of generating a model representation of the target joint ***30*** to the step of selecting a suitable joint replacement implant ***50*** as shown by the arrow ***32*.** Additionally, following selection of suitable joint replacement implant ***50***, the steps of obtaining measurement of target joint ***10*,** generating model representation of target joint ***30*** and generating projected model ***40*,** can be repeated in series or parallel as shown by the flow ***24, 25, 26*.**

FIG. **1B** is an alternate flow chart showing steps taken by a practitioner in assessing a joint. First, a practitioner obtains a measurement of a target joint ***10***. The step of obtaining a measurement can be accomplished by taking an image of the joint. This step can be repeated, as necessary, ***11*** to obtain a plurality of images in order to further refine the joint assessment process. Once the practitioner has obtained the necessary measurements, the information is used to generate a model representation of the target joint being assessed ***30***. This model representation can be in the form of a topographical map or image. The model representation of the joint can be in one, two, or three dimensions. The process can be repeated ***31*** as necessary or desired. It can include a physical model. After the model representation of the joint is assessed ***30***, the practitioner can optionally generate a projected model representation of the target joint in a corrected condition ***40*.** This step can be repeated ***41*** as necessary or desired. Using the difference between the topographical condition of the joint and the projected image of the joint, the practitioner can then design a joint implant ***52*** that is suitable to achieve the corrected joint anatomy, repeating the design process ***53*** as often as necessary to achieve the desired implant design. The practitioner can also assess whether providing additional features, such as rails, keels, lips, pegs, cruciate stems, or anchors, cross-bars, etc. will enhance the implants' performance in the target joint.

As will be appreciated by those of skill in the art, the practitioner can proceed directly from the step of generating a model representation of the target joint ***30*** to the step of designing a suitable joint replacement implant ***52*** as shown by the arrow ***38.*** Similar to the flow shown above, following the design of a suitable joint replacement implant ***52,*** the steps of obtaining measurement of target joint ***10,*** generating model representation of target joint ***30*** and generating projected model ***40***, can be repeated in series or parallel as shown by the flow ***42, 43, 44***.

**FIG. 1c** is a flow chart illustrating the process of selecting an implant for a patient. First, using the techniques described above or those suitable and known in the art at the time the invention is practiced, the size of area of diseased cartilage or cartilage loss is measured ***100.*** This step can be repeated multiple times ***101,*** as desired. Once the size of the cartilage defect is measured, the thickness of adjacent cartilage can optionally be measured ***110.*** This process can also be repeated as desired ***111.*** Either after measuring the cartilage loss or measuring the thickness of adjacent cartilage, the curvature of the articular surface is then measured ***120.*** Alternatively, the subchondral bone can be measured. As will be appreciated measurements can be taken of the surface of the joint being repaired, or of the mating surface in order to facilitate development of the best design for the implant surface.

Once the surfaces have been measured, the user either selects the best fitting implant contained in a library of implants ***130*** or generates a patient-specific implant ***132.*** These steps can be repeated as desired or necessary to achieve the best fitting implant for a patient, ***131, 133.*** As will be appreciated by those of skill in the art, the process of selecting or designing an implant can be tested against the information contained in the MRI or x-ray of the patient to ensure that the surfaces of the device achieves a good fit relative to the patient's joint surface. Testing can be accomplished by, for example, superimposing the implant image over the image for the patient's joint. Once it has been determined that a suitable implant has been selected or designed, the implant site can be prepared ***140,*** for example by removing cartilage or bone from the joint surface, or the implant can be placed into the joint ***150*.**

The joint implant selected or designed achieves anatomic or near anatomic fit with the existing surface of the joint while presenting a mating surface for the opposing joint surface that replicates the natural joint anatomy. In this instance, both the existing surface of the joint can be assessed as well as the desired resulting surface of the joint. This technique is particularly useful for implants that are not anchored into the bone.

As will be appreciated by those of skill in the art, the physician, or other person practicing the invention, can obtain a measurement of a target joint ***10*** and then either design ***52*** or select ***50*** a suitable joint replacement implant.

### II. REPAIR MATERIALS

A wide variety of materials find use in the practice of the present invention, including, but not limited to, plastics, metals, crystal free metals, ceramics, biological materials (*e.g.,* collagen or other extracellular matrix materials), hydroxyapatite, cells (*e.g*., stem cells, chondrocyte cells or the like), or combinations thereof. Based on the information (*e.g*., measurements) obtained regarding the defect and the articular surface and/or the subchondral bone, a repair material can be formed or selected. Further, using one or more of these techniques described herein, a cartilage replacement or regenerating material having a curvature that will fit into a particular cartilage defect, will follow the contour and shape of the articular surface, and will match the thickness of the surrounding cartilage. The repair material can include any combination of materials, and typically includes at least one non-pliable material, for example materials that are not easily bent or changed.

### A. METAL AND POLYMERIC REPAIR MATERIALS

Currently, joint repair systems often employ metal and/or polymeric materials including, for example, prostheses which are anchored into the underlying bone (*e.g.,* a femur in the case of a knee prosthesis). See, e.g., U.S. Patent No. 6,203,576 to Afriat, et al. issued March 20, 2001 and 6,322,588 to Ogle, et al. issued November 27, 2001, and references cited therein. A wide-variety of metals are useful in the practice of the present invention, and can be selected based on any criteria. For example, material selection can be based on resiliency to impart a desired degree of rigidity. Non-limiting examples of suitable metals include silver, gold, platinum, palladium, iridium, copper, tin, lead, antimony, bismuth, zinc, titanium, cobalt, stainless steel, nickel, iron alloys, cobalt alloys, such as Elgiloy®, a cobalt-chromium-nickel alloy, and MP35N, a nickel-cobalt-chromium-molybdenum alloy, and Nitinol™, a nickel-titanium alloy, aluminum, manganese, iron, tantalum, crystal free metals, such as Liquidmetal® alloys (available from LiquidMetal Technologies, www.liquidmetal.com), other metals that can slowly form polyvalent metal ions, for example to inhibit calcification of implanted substrates in contact with a patient's bodily fluids or tissues, and combinations thereof.

Suitable synthetic polymers include, without limitation, polyamides (*e.g*., nylon), polyesters, polystyrenes, polyacrylates, vinyl polymers (*e.g*., polyethylene, polytetrafluoroethylene, polypropylene and polyvinyl chloride), polycarbonates, polyurethanes, poly dimethyl siloxanes, cellulose acetates, polymethyl methacrylates, polyether ether ketones, ethylene vinyl acetates, polysulfones, nitrocelluloses, similar copolymers and mixtures thereof. Bioresorbable synthetic polymers can also be used such as dextran, hydroxyethyl starch, derivatives of gelatin, polyvinylpyrrolidone, polyvinyl alcohol, poly[N-(2-hydroxypropyl) methacrylamide], poly(hydroxy acids), poly(epsilon-caprolactone), polylactic acid, polyglycolic acid, poly(dimethyl glycolic acid), poly(hydroxy butyrate), and similar copolymers can also be used.

Other materials would also be appropriate, for example, the polyketone known as polyetheretherketone (PEEK™). This includes the material PEEK 450G, which is an unfilled PEEK approved for medical implantation available from Victrex of Lancashire, Great Britain. (Victrex is located at www.matweb.com or see Boedeker www.boedeker.com). Other sources of this material include Gharda located in Panoli, India (www.ghardapolymers.com).

It should be noted that the material selected can also be filled. For example, other grades of PEEK are also available and contemplated, such as 30% glass-filled or 30% carbon filled, provided such materials are cleared for use in implantable devices by the FDA, or other regulatory body. Glass filled PEEK reduces the expansion rate and increases the flexural modulus of PEEK relative to that portion which is unfilled. The resulting product is known to be ideal for improved strength, stiffness, or stability. Carbon filled PEEK is known to enhance the compressive strength and stiffness of PEEK and lower its expansion rate. Carbon filled PEEK offers wear resistance and load carrying capability.

As will be appreciated, other suitable similarly biocompatible thermoplastic or thermoplastic polycondensate materials that resist fatigue, have good memory, are flexible, and/or deflectable have very low moisture absorption, and good wear and/or abrasion resistance, can be used without departing from the scope of the invention. The implant can also be comprised of polyetherketoneketone (PEKK).

Other materials that can be used include polyetherketone (PEK), polyetherketoneetherketoneketone (PEKEKK), and polyetheretherketoneketone (PEEKK), and generally a polyaryletheretherketone. Further other polyketones can be used as well as other thermoplastics.

Reference to appropriate polymers that can be used for the implant can be made to the following documents, all of which are incorporated herein by reference. These documents include: PCT Publication WO 02/02158 A1, dated Jan. 10, 2002 and entitled Bio-Compatible Polymeric Materials; PCT Publication WO 02/00275 A1, dated Jan. 3, 2002 and entitled Bio-Compatible Polymeric Materials; and PCT Publication WO 02/00270 A1, dated Jan. 3, 2002 and entitled Bio-Compatible Polymeric Materials.

The polymers can be prepared by any of a variety of approaches including conventional polymer processing methods. Preferred approaches include, for example, injection molding, which is suitable for the production of polymer components with significant structural features, and rapid prototyping approaches, such as reaction injection molding and stereo-lithography. The substrate can be textured or made porous by either physical abrasion or chemical alteration to facilitate incorporation of the metal coating. Other processes are also appropriate, such as extrusion, injection, compression molding and/or machining techniques. Typically, the polymer is chosen for its physical and mechanical properties and is suitable for carrying and spreading the physical load between the joint surfaces.

More than one metal and/or polymer can be used in combination with each other. For example, one or more metal-containing substrates can be coated with polymers in one or more regions or, alternatively, one or more polymer-containing substrate can be coated in one or more regions with one or more metals.

The system or prosthesis can be porous or porous coated. The porous surface components can be made of various materials including metals, ceramics, and polymers. These surface components can, in turn, be secured by various means to a multitude of structural cores formed of various metals. Suitable porous coatings include, but are not limited to, metal, ceramic, polymeric (*e.g*., biologically neutral elastomers such as silicone rubber, polyethylene terephthalate and/or combinations thereof) or combinations thereof. See, *e.g*., U.S. Pat. No. 3,605,123 to Hahn, issued September 20, 1971. U.S. Pat. No. 3,808,606 to Tronzo issued May 7, 1974 and U.S. Pat. No. 3,843,975 to Tronzo issued October 29, 1974; U.S. Pat. No. 3,314,420 to Smith issued April 18, 1967; U.S. Pat. No. 3,987,499 to Scharbach issued October 26, 1976; and German Offenlegungsschrift 2,306,552. There can be more than one coating layer and the layers can have the same or different porosities. See, *e.g*., U.S. Pat. No. 3,938,198 to Kahn, et al., issued February 17, 1976.

The coating can be applied by surrounding a core with powdered polymer and heating until cured to form a coating with an internal network of interconnected pores. The tortuosity of the pores (*e.g*., a measure of length to diameter of the paths through the pores) can be important in evaluating the probable success of such a coating in use on a prosthetic device. See, also, U.S. Pat. No. 4,213,816 to Morris issued July 22, 1980. The porous coating can be applied in the form of a powder and the article as a whole subjected to an elevated temperature that bonds the powder to the substrate. Selection of suitable polymers and/or powder coatings can be determined in view of the teachings and references cited herein, for example based on the melt index of each.

### B. BIOLOGICAL REPAIR MATERIAL

Repair materials can also include one or more biological material either alone or in combination with non-biological materials. For example, any base material can be designed or shaped and suitable cartilage replacement or regenerating material(s) such as fetal cartilage cells can be applied to be the base. The cells can be then be grown in conjunction with the base until the thickness (and/or curvature) of the cartilage surrounding the cartilage defect has been reached. Conditions for growing cells (*e.g*., chondrocytes) on various substrates in culture, ex *vivo* and *in vivo* are described, for example, in U.S. Patent Nos. 5,478,739 to Slivka et al. issued December 26, 1995; 5,842,477 to Naughton et al. issued December 1, 1998; 6,283,980 to Vibe-Hansen et al., issued September 4, 2001, and 6,365,405 to Salzmann et al. issued April 2, 2002. Non-limiting examples of suitable substrates include plastic, tissue scaffold, a bone replacement material (*e.g*., a hydroxyapatite, a bioresorbable material), or any other material suitable for growing a cartilage replacement or regenerating material on it.

Biological polymers can be naturally occurring or produced *in vitro* by fermentation and the like. Suitable biological polymers include, without limitation, collagen, elastin, silk, keratin, gelatin, polyamino acids, cat gut sutures, polysaccharides (*e.g*., cellulose and starch) and mixtures thereof. Biological polymers can be bioresorbable.

Biological materials used in the methods described herein can be autografts (from the same subject); allografts (from another individual of the same species) and/or xenografts (from another species). See, also, International Patent Publications WO 02/22014 to Alexander et al. published March 21, 2002 and WO 97/27885 to Lee published August 7, 1997. In certain embodiments autologous materials are preferred, as they can carry a reduced risk of immunological complications to the host, including re-absorption of the materials, inflammation and/or scarring of the tissues surrounding the implant site.

In one example, a probe is used to harvest tissue from a donor site and to prepare a recipient site. The donor site can be located in a xenograft, an allograft or an autograft. The probe is used to achieve a good anatomic match between the donor tissue sample and the recipient site. The probe is specifically designed to achieve a seamless or near seamless match between the donor tissue sample and the recipient site. The probe can, for example, be cylindrical. The distal end of the probe is typically sharp in order to facilitate tissue penetration. Additionally, the distal end of the probe is typically hollow in order to accept the tissue. The probe can have an edge at a defined distance from its distal end, *e.g*. at 1 cm distance from the distal end and the edge can be used to achieve a defined depth of tissue penetration for harvesting. The edge can be external or can be inside the hollow portion of the probe. For example, an orthopedic surgeon can take the probe and advance it with physical pressure into the cartilage, the subchondral bone and the underlying marrow in the case of a joint such as a knee joint. The surgeon can advance the probe until the external or internal edge reaches the cartilage surface. At that point, the edge will prevent further tissue penetration thereby achieving a constant and reproducible tissue penetration. The distal end of the probe can include one or more blades, saw-like structures, or tissue cutting mechanism. For example, the distal end of the probe can include an iris-like mechanism consisting of several small blades. The blade or blades can be moved using a manual, motorized or electrical mechanism thereby cutting through the tissue and separating the tissue sample from the underlying tissue. Typically, this will be repeated in the donor and the recipient. In the case of an iris-shaped blade mechanism, the individual blades can be moved so as to close the iris thereby separating the tissue sample from the donor site.

In another example, a laser device or a radiofrequency device can be integrated inside the distal end of the probe. The laser device or the radiofrequency device can be used to cut through the tissue and to separate the tissue sample from the underlying tissue.

In one example, the same probe can be used in the donor and in the recipient. In another embodiment, similarly shaped probes of slightly different physical dimensions can be used. For example, the probe used in the recipient can be slightly smaller than that used in the donor thereby achieving a tight fit between the tissue sample or tissue transplant and the recipient site. The probe used in the recipient can also be slightly shorter than that used in the donor thereby correcting for any tissue lost during the separation or cutting of the tissue sample from the underlying tissue in the donor material.

Any biological repair material can be sterilized to inactivate biological contaminants such as bacteria, viruses, yeasts, molds, mycoplasmas and parasites. Sterilization can be performed using any suitable technique, for example radiation, such as gamma radiation.

Any of the biological materials described herein can be harvested with use of a robotic device. The robotic device can use information from an electronic image for tissue harvesting.

In certain examples, the cartilage replacement material has a particular biochemical composition. For instance, the biochemical composition of the cartilage surrounding a defect can be assessed by taking tissue samples and chemical analysis or by imaging techniques. For example, WO 02/22014 to Alexander describes the use of gadolinium for imaging of articular cartilage to monitor glycosaminoglycan content within the cartilage. The cartilage replacement or regenerating material can then be made or cultured in a manner, to achieve a biochemical composition similar to that of the cartilage surrounding the implantation site. The culture conditions used to achieve the desired biochemical compositions can include, for example, varying concentrations. Biochemical composition of the cartilage replacement or regenerating material can, for example, be influenced by controlling concentrations and exposure times of certain nutrients and growth factors.

### III. DEVICE DESIGN

### A. CARTILAGE MODELS

Using information on thickness and curvature of the cartilage, a physical model of the surfaces of the articular cartilage and of the underlying bone can be created. This physical model can be representative of a limited area within the joint or it can encompass the entire joint. This model can also take into consideration the presence or absence of a meniscus as well as the presence or absence of some or all of the cartilage. For example, in the knee joint, the physical model can encompass only the medial or lateral femoral condyle, both femoral condyles and the notch region, the medial tibial plateau, the lateral tibial plateau, the entire tibial plateau, the medial patella, the lateral patella, the entire patella or the entire joint. The location of a diseased area of cartilage can be determined, for example using a 3D coordinate system or a 3D Euclidian distance as described in WO 02/22014.

In this way, the size of the defect to be repaired can be determined. This process takes into account that, for example, roughly 80% of patients have a healthy lateral component. As will be apparent, some, but not all, defects will include less than the entire cartilage. Thus, in one method, the thickness of the normal or only mildly diseased cartilage surrounding one or more cartilage defects is measured. This thickness measurement can be obtained at a single point or, preferably, at multiple points, for example 2 point, 4-6 points, 7-10 points, more than 10 points or over the length of the entire remaining cartilage. Furthermore, once the size of the defect is determined, an appropriate therapy (*e.g*., articular repair system) can be selected such that as much as possible of the healthy, surrounding tissue is preserved.

In other methods, the curvature of the articular surface can be measured to design and/or shape the repair material. Further, both the thickness of the remaining cartilage and the curvature of the articular surface can be measured to design and/or shape the repair material. Alternatively, the curvature of the subchondral bone can be measured and the resultant measurement(s) can be used to either select or shape a cartilage replacement material. For example, the contour of the subchondral bone can be used to re-create a virtual cartilage surface: the margins of an area of diseased cartilage can be identified. The subchondral bone shape in the diseased areas can be measured. A virtual contour can then be created by copying the subchondral bone surface into the cartilage surface, whereby the copy of the subchondral bone surface connects the margins of the area of diseased cartilage. In shaping the device, the contours can be configured to mate with existing cartilage or to account for the removal of some or all of the cartilage.

**FIG. 2A** shows a slightly perspective top view of a joint implant ***200*** of the invention suitable for implantation at the tibial plateau of the knee joint. As shown in **FIG. 2A****,** the implant can be generated using, for example, a dual surface assessment, as described above with respect to **FIGS. 1A** and **B**.

The implant ***200*** has an upper surface ***202,*** a lower surface ***204*** and a peripheral edge ***206.*** The upper surface ***202*** is formed so that it forms a mating surface for receiving the opposing joint surface; in this instance partially concave to receive the femur. The concave surface can be variably concave such that it presents a surface to the opposing joint surface, e.g. a negative surface of the mating surface of the femur it communicates with. As will be appreciated by those of skill in the art, the negative impression, need not be a perfect one.

The upper surface ***202*** of the implant ***200*** can be shaped by any of a variety of means. For example, the upper surface ***202*** can be shaped by projecting the surface from the existing cartilage and/or bone surfaces on the tibial plateau, or it can be shaped to mirror the femoral condyle in order to optimize the complimentary surface of the implant when it engages the femoral condyle. Alternatively, the superior surface *202* can be configured to mate with an inferior surface of an implant configured for the opposing femoral condyle.

The lower surface ***204*** has a convex surface that matches, or nearly matches, the tibial plateau of the joint such that it creates an anatomic or near anatomic fit with the tibial plateau. Depending on the shape of the tibial plateau, the lower surface can be partially convex as well. Thus, the lower surface ***204*** presents a surface to the tibial plateau that fits within the existing surface. It can be formed to match the existing surface or to match the surface after articular resurfacing.

As will be appreciated by those of skill in the art, the convex surface of the lower surface ***204*** need not be perfectly convex. Rather, the lower surface ***204*** is more likely consist of convex and concave portions that fit within the existing surface of the tibial plateau or the re-surfaced plateau. Thus, the surface is essentially variably convex and concave.

**FIG. 2B** shows a top view of the joint implant of **FIG. 2A****.** As shown in **FIG. 2B** the exterior shape ***208*** of the implant can be elongated. The elongated form can take a variety of shapes including elliptical, quasi-elliptical, race-track, etc. However, as will be appreciated the exterior dimension is typically irregular thus not forming a true geometric shape, *e.g.* ellipse. As will be appreciated by those of skill in the art, the actual exterior shape of an implant can vary depending on the nature of the joint defect to be corrected. Thus the ratio of the length **L** to the width ***W*** can vary from, for example, between 0.25 to 2.0, and more specifically from 0.5 to 1.5. As further shown in **FIG. 2B****,** the length across an axis of the implant ***200*** varies when taken at points along the width of the implant. For example, as shown in **FIG. 2B****, L₁ ≠ L₂ ≠ L₃.**

Turning now to **FIGS. 2C-E,** cross-sections of the implant shown in **FIG. 2B** are depicted along the lines of C-C, D-D, and E-E. The implant has a thickness ***t1, t2*** and ***t3*** respectively. As illustrated by the cross-sections, the thickness of the implant varies along both its length ***L*** and width ***W***. The actual thickness at a particular location of the implant ***200*** is a function of the thickness of the cartilage and/or bone to be replaced and the joint mating surface to be replicated. Further, the profile of the implant ***200*** at any location along its length ***L*** or width ***W*** is a function of the cartilage and/or bone to be replaced.

**FIG. 2F** is a lateral view of the implant ***200*** of **FIG. 2A****.** In this instance, the height of the implant ***200*** at a first end ***h₁*** is different than the height of the implant at a second end ***h₂***. Further the upper edge ***208*** can have an overall slope in a downward direction. However, as illustrated the actual slope of the upper edge ***208*** varies along its length and can, in some instances, be a positive slope. Further the lower edge ***210*** can have an overall slope in a downward direction. As illustrated the actual slope of the lower edge ***210*** varies along its length and can, in some instances, be a positive slope. As will be appreciated by those of skill in the art, depending on the anatomy of an individual patient, an implant can be created wherein ***h₁*** and ***h₂*** are equivalent, or substantially equivalent without departing from the scope of the invention.

**FIG. 2G** is a cross-section taken along a sagittal plane in a body showing the implant ***200*** implanted within a knee joint ***1020*** such that the lower surface ***204*** of the implant ***200*** lies on the tibial plateau ***1022*** and the femur ***1024*** rests on the upper surface ***202*** of the implant ***200***. **FIG. 2H** is a cross-section taken along a coronal plane in a body showing the implant ***200*** implanted within a knee joint ***1020.*** As is apparent from this view, the implant ***200*** is positioned so that it fits within a superior articular surface ***224.*** As will be appreciated by those of skill in the art, the articular surface could be the medial or lateral facet, as needed.

**FIG. 2I** is a view along an axial plane of the body showing the implant ***200*** implanted within a knee joint ***1020*** showing the view taken from an aerial, or upper, view. **FIG. 2J** is a view of an alternate example where the implant is a bit larger such that it extends closer to the bone medially, *i.e.* towards the edge *1023* of the tibial plateau, as well as extending anteriorly and posteriorly.

**FIG. 2K** is a cross-section of an implant ***200***, wherein the lower surface ***204*** further includes a joint anchor ***212*.** As illustrated in this example, the joint anchor **212** forms a protrusion, keel or vertical member that extends from the lower surface ***204*** of the implant **200** and projects into, for example, the bone of the joint. As will be appreciated by those of skill in the art, the keel can be perpendicular or lie within a plane of the body.

Additionally, as shown in **FIG. 2L** the joint anchor **212** can have a cross-member ***214*** so that from a bottom perspective, the joint anchor ***212*** has the appearance of a cross or an "x." As will be appreciated by those of skill in the art, the joint anchor ***212*** could take on a variety of other forms while still accomplishing the same objective of providing increased stability of the implant ***200*** in the joint. These forms include, but are not limited to, pins, bulbs, balls, teeth, etc. Additionally, one or more joint anchors ***212*** can be provided as desired. **FIG. 2M** and **N** illustrate cross-sections of alternate embodiments of a dual component implant from a side view and a front view.

In an alternate example shown in **FIG. 2M** it may be desirable to provide a one or more cross-members *220* on the lower surface ***204*** in order to provide a bit of translation movement of the implant relative to the surface of the femur, or femur implant. In that event, the cross-member can be formed integral to the surface of the implant or can be one or more separate pieces that fit within a groove ***222*** on the lower surface ***204*** of the implant ***200.*** The groove can form a single channel as shown in **FIG. 2N1,** or can have more than one channel as shown in **FIG**. **2N2.** In either event, the cross-bar then fits within the channel as shown in **FIGS. 2N1-N2.** The cross-bar members ***220*** can form a solid or hollow tube or pipe structure as shown in **FIG. 2P****.** Where two, or more, tubes ***220*** communicate to provide translation, a groove ***221*** can be provided along the surface of one or both cross-members to interlock the tubes into a cross-bar member further stabilizing the motion of the cross-bar relative to the implant ***200*.** As will be appreciated by those of skill in the art, the cross-bar member ***220*** can be formed integrally with the implant.

As shown in **FIGS. 2Q-R,** it is anticipated that the surface of the tibial plateau will be prepared by forming channels thereon to receive the cross-bar members. Thus facilitating the ability of the implant to seat securely within the joint while still providing movement about an axis when the knee joint is in motion.

**FIG. 2S(1-9)** illustrate an alternate example of implant ***200*.** As illustrated in **FIG. 2s** the edges are beveled to relax a sharp corner. **FIG. 2S(1)** illustrates an implant having a single fillet or bevel ***230.*** The fillet is placed on the implant anterior to the posterior portion of the tibial spine. As shown in **FIG. 2S(2)** two fillets ***230, 231*** are provided and used for the posterior chamfer. In **FIG. 2S(3)** a third fillet ***234*** is provided to create two cut surfaces for the posterior chamfer.

Turning now to **FIG. 2S(4)** a tangent of the implant is deselected, leaving three posterior curves. **FIG. 2S(5)** shows the result of tangent propagation. **FIG. 2S(6)** illustrates the effect on the design when the bottom curve is selected without tangent propagation. The result of tangent propagation and selection is shown in **FIG. 2S(7).** As can be seen in **FIG. 2S(8-9)** the resulting corner has a softer edge but sacrifices less than 0.5 mm of joint space. As will be appreciated by those of skill in the art, additional cutting planes can be added without departing from the scope of the invention.

**FIG. 2T** illustrates an alternate example of an implant ***200*** wherein the surface of the tibial plateau ***250*** is altered to accommodate the implant. As illustrated in **FIG. 2T(1-2)** the tibial plateau can be altered for only half of the joint surface ***251*** or for the full surface ***252.*** As illustrate in **FIG. 2T(3-4)** the posterior-anterior surface can be flat ***260*** or graded ***262.*** Grading can be either positive or negative relative to the anterior surface. Grading can also be used with respect to the implants of **FIG. 2T** where the grading either lies within a plane or a body or is angled relative to a plane of the body. Additionally, attachment mechanisms can be provided to anchor the implant to the altered surface. As shown in **FIG. 2T(5-7)** keels ***264*** can be provided. The keels ***264*** can either sit within a plane, e.g. sagittal or coronal plane, or not sit within a plane (as shown in **FIG. 2T(7)**)**. FIG. 2T(8)** illustrates an implant which covers the entire tibial plateau. The upper surface of these implants are designed to conform to the projected shape of the joint as determined under the steps described with respect to FIG. 1, while the lower surface is designed to be flat, or substantially flat to correspond to the modified surface of the joint.

Turning now to **FIGS. 3A-I** an implant suitable for providing an opposing joint surface to the implant of **FIG. 2A** is shown. This implant corrects a defect on an inferior surface of the femur ***1024** (e.g.,* the condyle of the femur that mates with the tibial plateau) and can be used alone, *i.e*., on the femur ***1024,*** or in combination with another joint repair device. Formation of the surfaces of the devices can be achieved using the techniques described above with respect to the implant of **FIG. 2****.**

**FIG. 3A** shows a perspective view of an implant ***300*** having a curved mating surface ***302*** and convex joint abutting surface *304.* The joint abutting surface ***304*** need not form an anatomic or near anatomic fit with the femur in view of the anchors ***306*** provided to facilitate connection of the implant to the bone. In this instance, the anchors ***306*** are shown as pegs having notched heads. The notches facilitate the anchoring process within the bone. However, pegs without notches can be used as well as pegs with other configurations that facilitate the anchoring process or cruciate stems. Pegs and other portions of the implant can be porous coated. The implant can be inserted without bone cement or with use of bone cement. The implant can be designed to abut the subchondral bone, *i.e.* it can substantially follow the contour of the subchondral bone. This has the advantage that no bone needs to be removed other than for the placement of the peg holes thereby significantly preserving bone stock.

The anchors ***306*** could take on a variety of other forms while still accomplishing the same objective of providing increased stability of the implant ***300*** in the joint. These forms include, but are not limited to, pins, bulbs, balls, teeth, etc. Additionally, one or more joint anchors ***306*** can be provided as desired. As illustrated in **FIG. 3****,** three pins are used to anchor the implant ***300.*** However, more or fewer joint anchors, cruciate stems, or pins, can be used .

**FIG. 3B** shows a slightly perspective superior view of the bone mating surface ***304*** further illustrating the use of three anchors ***306*** to anchor the implant to the bone. Each anchor ***306*** has a stem ***310*** with a head ***312*** on top. As shown in **FIG. 3c****,** the stem ***310*** has parallel walls such that it forms a tube or cylinder that extends from the bone mating surface ***304.*** A section of the stem forms a narrowed neck ***314*** proximal to the head ***312.*** As will be appreciated by those of skill in the art, the walls need not be parallel, but rather can be sloped to be shaped like a cone. Additionally, the neck ***314*** need not be present, nor the head ***312.*** As discussed above, other configurations suitable for anchoring can be used.

Turning now to **FIG. 3D****,** a view of the tibial plateau mating surface ***302*** of the implant ***300*** is illustrated. As is apparent from this view, the surface is curved such that it is convex or substantially convex in order to mate with the concave surface of the plateau. **FIG. 3E** illustrates the upper surface ***304*** of the implant ***300*** further illustrating the use of three pegs ***306*** for anchoring the implant ***300*** to the bone. As illustrated, the three pegs ***306*** are positioned to form a triangle. However, as will be appreciated by those of skill in the art, one or more pegs can be used, and the orientation of the pegs ***306*** to one another can be as shown, or any other suitable orientation that enables the desired anchoring. **FIG. 3F** illustrated a cross section of the implant *300* taken along the lines F-F shown in **FIG. 3E**. Typically the pegs are oriented on the surface of the implant so that the peg is perpendicular to the femoral condyle, which may not result in the peg being perpendicular to the surface of the implant.

**FIG. 3G** illustrates the axial view of the femur ***1000*** having a lateral condyle ***1002*** and a medial condyle ***1004.*** The intercondylar fossa is also shown ***1006*** along with the lateral epicondyle ***1008*** and medial epicondyle ***1010.*** Also shown is the patellar surface of the femur ***1012.*** The implant ***300*** illustrated in **FIG. 3A****,** is illustrated covering a portion of the lateral condyle. The pegs ***306*** are also shown that facilitate anchoring the implant ***300*** to the condyle.

**FIG. 3H** illustrates a knee joint ***1020*** from an anterior perspective. The implant ***300*** is implanted over a condyle. As shown in **FIG. 3I** the implant ***300*** is positioned such that it communicates with an implant ***200*** designed to correct a defect in the tibial plateau, such as those shown in **FIGS. 2****.**

**FIGS. 3J-K** illustrate an implant ***300*** for placement on a condyle. In this example, at least one flat surface or chamfer cut ***360*** is provided to mate with a cut made on the surface of the condyle in preparing the joint. The flat surface ***360*** typically does not encompass the entire proximal surface ***304*** of the implant ***300.***

**FIG. 4A** illustrates the design of a typical total knee arthroplasty ("TKA") primary knee ***499 .*** Posterior cuts ***498,*** anterior cuts ***497*** and distal cuts ***496*** are provided as well as chamfer cuts ***495.***

**FIGS. 4B** and **4c** illustrate another implant ***400.*** As shown in **FIG. 4B****,** the implant ***400*** is configured such that it covers both the lateral and medial femoral condyle along with the patellar surface of the femur *1012.* The implant ***400*** has a lateral condyle component *410* and a medial condyle component ***420*** and a bridge ***430*** that connects the lateral condyle component *410* to the medial condyle component ***420*** while covering at least a portion of the patellar surface of the femur *1012.* The implant ***400*** can optionally oppose one or more implants, such as those shown in **FIG. 2****,** if desired. **FIG. 4c** is a side view of the implant of **FIG. 4B****.** As shown in **FIG. 4c****,** the superior surface ***402*** of the implant ***400*** is curved to correspond to the curvature of the femoral condyles. The curvature can be configured such that it corresponds to the actual curvature of one or both of the existing femoral condyles, or to the curvature of one or both of the femoral condyles after resurfacing of the joint. One or more pegs ***430*** can be provided to assist in anchoring the implant to the bone. As will be appreciated by those of skill in the art, the implant can be configured such that the superior surface contacting a first condyle is configured to male with the existing condule while a surface contacting a second condyle has one or more flat surfaces to mate with a condyle surface that has been modified.

**FIG. 4D** illustrates a top view of the implant ***400*** shown in **FIG. 4B****.** As is should be appreciated from this view, the inferior surface ***404*** of the implant ***400*** is configured to conform to the shape of the femoral condyles, e.g. the shape healthy femoral condyles would present to the tibial surface in a non-damaged joint.

**FIGS. 4E** and **F** illustrate perspective views of the implant from the inferior surface (*i.e.,* tibial plateau mating surface).

**FIG. 4G** illustrates the axial view of the femur ***1000*** having a lateral condyle ***1002*** and a medial condyle *1004.* The intercondylar fossa is also shown ***1006*** along with the lateral epicondyle ***1008.*** The implant ***400*** illustrated in **FIG. 4B****,** is illustrated covering both condyles and the patellar surface of the femur *1012.* The pegs *430* are also shown that facilitate anchoring the implant ***400*** to the condyle.

**FIG. 4H** illustrates a knee joint *1050* from an anterior perspective. The implant ***400*** is implanted over both condyles. As shown in **FIG. 4I** the implant ***400*** is positioned such that it communicates with an implant ***200*** designed to correct a defect in the tibial plateau, such as those shown in **FIGS. 2****.**

As will be appreciated by those of skill in the art, the implant ***400*** can be manufactured from a material that has memory such that the implant can be configured to snap-fit over the condyle. Alternatively, it can be shaped such that it conforms to the surface without the need of a snap-fit.

**FIGS. 5A** and **5B** illustrate yet another implant according to the invention that suitable for repairing a damaged condyle. As shown in **FIG. 5A****,** the implant *500* is configured such that it covers only one of the lateral or medial femoral condyles ***510.*** The implant differs from the implant of **FIG. 3** in that the implant ***500*** also covers at least a portion of the patellar surface of the femur ***512.***

Similar to the implant of **FIG. 4****,** the implant can optionally oppose one or more implants or opposing joint surfaces, such as those shown in **FIG. 2****,** and can be combined with other implants, such as the implants of **FIG. 3****.** **FIG. 5c** is a perspective side view of the implant of **FIG. 5A****.** As shown in **FIG.5C****,** the superior surface ***502*** of the implant *500* is curved to correspond to the curvature of the femoral condyle that it mates with and the portion of the patellar surface of the femur that it covers. One or more pegs ***530*** can be provided to assist in anchoring the implant to the bone. Additionally, an angled surface ***503*** can be provided on an interior surface ***502*** of the condyle component that conforms to an optionally provided cut made on the surface of the joint surface with which the implant mates.

**FIG. 5D** illustrates a perspective top view of the implant ***500*** shown in **FIG. 5A****.** As is should be appreciated from this view, the inferior surface ***504*** of the implant ***500*** is configured to conform to the projected shape of the femoral condyles, e.g. the shape healthy femoral condyles would present to the tibial surface in a non-damaged joint.

**FIG. 5E** is a view of the implant ***500*** showing a hatched three point loading support area which extends from a top portion ***513*** to a line (plane 17) and from a line (plane 18) to a bottom portion ***515.*** Also illustrated are the pegs ***530*** extending from the superior surface. **FIG. 5F** illustrates the superior surface of the implant ***500*** with the pegs ***530*** extending from the superior surface. **FIG. 5F** also illustrates the hatched cantilever loading support area, which extends from the line (plane 18) to the top portion ***513*** of the implant. The loading forces and directions for each support condition are based on physiological load encounters. Table 1 shows the Physiological Loadings taken from a study by Seth Greenwald

**Table 1**

| **Physiological Loadings¹** | | | |
|---|---|---|---|
| **Set-up** | **"1"** | **"2"** | **"3"** |
| **Flexion Angle (degree)** | 0° | 60° | 90° |
| **Normal Force N** | 2,900 | 3,263 | 3,625 |
| **(Ibs.)** | (652) | (733.5) | (815) |
| **Normal Force Case** | Walking (4.0 x BW^{¥}) | Stair Descent (4.5 x BW^{¥}) | Stair Ascent (5.0 x BW^{¥}) |

| | | | |
|---|---|---|---|
| ^{¥}Body Weight (BW) taken as a 60 year old male, with 173 cm height for an average body weight of 74 kg (163 Ibs). ¹"Tibial Plateau Surface Stress in TKA: A Factor Influencing Polymer Failure Series III - Posterior Stabilized Designs;" Paul D. Postak, B.Sc., Christine S. Heim, B.Sc., A. Seth Greenwald, D. Phil.; Orthopaedic Research Laboratories, The Mt. Sinai Medical Center, Cleveland, Ohio. Presented at the 62nd Annual AAOS Meeting, 1995. | | | |

Using the implant ***500*** described in this application, the three point loading will occur from set-up 1 (2900 N). To replicate a worst case loading scenario, a 75/25 load distribution (75% of 2900 N = 2175 N) will be used. The loading will be concentrated on a 6mm diameter circular area located directly below and normal to the ped on the bearing surface.

Turning to the cantilever loading shown in FIG. 5F, the loading will occur from set-up 3, or 90°, at a 75/25 load distribution (75% of 3625 N = 2719 N). As with the above example, the loading will be concentrated on a 6 mm diameter circular area located at the center of the posterior-most portion of the medial condyle normal to the flat cut surface of the posterior condyle.

**FIGS. 5G** and **H** illustrate alternate embodiments of the implant ***500*** having a rail design that provides one or more rails ***521*** along medial and/or lateral sides of the implant ***500.*** The rail ***521*** can be positioned so that it extends along a portion of the medial ***517*** and/or lateral ***519*** sides before communicating with the angled surface ***503.*** As will be appreciate, a single side rail ***521***can be provided without departing from the scope of the invention.

**FIG. 5I** illustrates another embodiment of an implant *500* having a keel design. A keel ***523*** (or centrally formed rail) is provided on the superior surface of the implant. In this embodiment, the keel ***523*** is located on the surface of the implant, but not at the sides. As will be appreciated, the keel can be centered, as shown, substantially centered, or located off-center. An angled surface ***503*** can be provided to communicate with a modified joint surface. Alternatively, where the joint surface is worn or modified, the cut ***503*** could be configured to mate with the worn or modified surface.

**FIG. 5J** illustrates the axial view of the femur ***1000*** having a lateral condyle ***1002*** and a medial condyle *1004.* The intercondylar fossa is also shown *1006* along with the lateral epicondyle *1008* and the medial epicondyle *1010.* The patellar surface of the femur *1012* is also illustrated. The implant ***500,*** illustrated in **FIG. 5A****,** is shown covering the lateral condyle and a portion of the patellar surface of the femur *1012.* The pegs ***530*** are also shown that facilitate anchoring the implant ***500*** to the condyle and patellar surface.

**FIG. 5K** illustrates a knee joint *1020* from an anterior perspective. The implant ***500*** is implanted over the lateral condyle. **FIG. 5L** illustrates a knee joint *1020* with the implant ***500*** covering the medial condyle *1004.* As illustrated in **FIGS. 5K** and **L** the shape of the implant *500* corresponding to the patella surface can take on a variety of curvatures without departing from the scope of the invention.

Turning now to **FIG. 5M** and **N** the implant ***500*** is positioned such that it communicates with an implant ***200*** designed to correct a defect in the tibial plateau, such as those shown in **FIGS. 2****.**

In another embodiment of the invention, the implant ***500*** can have a superior surface ***502*** which substantially conforms to the surface of the condyle but which has at one flat portion corresponding to an oblique cut on the bone as shown in **FIG. 5o****.**

Turning now to **FIG. 5P-Q** an implant ***500*** is shown from a side view with a 7° difference between the anterior and posterior cuts.

**FIG. 5R-S** illustrate an implant ***500*** having a contoured surface ***560*** for mating with the joint surface and an anterior cut ***561*** and a posterior cut ***562.*** **FIG. 5S** shows the same implant ***500*** from a slightly different angle. **FIG. 5T** illustrates another implant ***500*** having a contoured surface ***560*** for mating with the joint surface and posterior cut ***562,*** a distal cut ***563,*** and a chamfer cut ***564.*** In this embodiment no anterior cut is provided. **FIG. 5U** illustrates the implant ***500*** of **FIG. 5T** from a side perspective. The cuts are typically less than the cut required for a TKA, i.e., typically less than 10mm. The design of the cuts for this implant allow for a revision surgery to the knee, if required, at a later date.

**FIGS. 6A-G** illustrate the implant ***500*** of **FIG. 5** with a graphical representation of the cross-sections ***610, 620*** from which a surface shape of the implant is derived. **FIG. 6A** illustrates a top view of the implant ***500*** sitting on top of the extracted surface shape ***600***. This view of the implant ***500*** illustrates a notch ***514*** associated with the bridge section of the implant ***512*** which covers the patellar surface of the femur (or the trochlea region) to provide a mating surface that approximates the cartilage surface. As will be appreciated by those of skill in the art, the shape of an implant designed for the medial condyle would not necessarily be a mirror image of the implant designed for the lateral condyle because of differences in anatomy. Thus, for example, the notch ***514*** would not be present in an implant designed for the medial condyle and the patellar surface of the femur. Therefore, the implant can be designed to include all or part of the troclea region or to exclude it entirely.

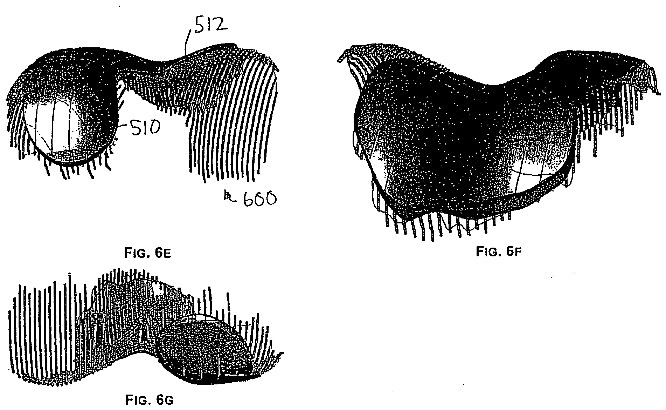

**FIG. 6B** illustrates a bottom view of the implant ***500*** layered over another derived surface shape ***601.*** **FIG. 6c** is a bottom view showing the implant ***500*** extending through the extracted surface shape ***600*** shown in **FIG. 6A. FIG. 6D** is a close-up view of the **FIG. 6c** showing the condylar wing of the implant covering the extracted surface ***600***. **FIG. 6E** illustrates a top posterior view of the implant ***500*** positioned over the graphical representation of the surface shape ***600***. **FIG. 6F** is an anterior view and **FIG. 6G** is a bottom-posterior view.

**FIG. 7A-C** illustrate an implant ***700*** for correcting a joint similar to the implant ***500*** above. However, implant ***700*** consists of two components. The first component ***710*** engages a condyle of the femur, either medial or lateral depending on the design. The second component ***720*** engages the patellar surface of the femur. As discussed with the previous embodiments, the surfaces of the implant ***700*** can be configured such that the distal surface ***722*** (e.g., the surface that faces the tibial plateau) is shaped based on a projection of the natural shape of the femur compensating the design for valgus or varus deformities and/or flattening of the surface of the femur. Alternatively, the distal surface can be shaped based on the shape of the tibial plateau to provide a surface designed to optimally mate with the tibial plateau. The proximal surface ***724*** (e.g., the surface that engages the femoral condyle) can be configured such that it mirrors the surface of the femur in either its damaged condition or its modified condition. Likewise, the proximal surface can have one or more flattened sections ***726*** that form, e.g., chamfer cuts. Additionally the surface can include mechanisms facilitating attachment ***728*** to the femur, such as keels, teeth, cruciate stems, and the like. The medial facing portion of the condyle implant has a tapered surface ***730*** while the lateral facing portion of the patellar component also has a tapered surface such that each component presents tapered surfaces ***730*** to the other component.

By dividing the surfaces of the medial and lateral compartments into independent articulating surfaces, as shown in **FIG. 7****,** the implant provides improved fit of the conformal surfaces to the subchondral bone. Additionally, the lateral-anterior portion of the femur is shielded from stress which could cause bone loss. Also, the smaller size of each component of the implant, enables the implant to be placed within the joint using a smaller incision. Finally, the wear of the patellar component is improved.

**FIGS. 8A-F** illustrate a patella ***00*** with an implants ***810.*** The implant ***810*** can have one or more pegs, cruciate stems, or other anchoring mechanisms, if desired. As will be appreciated by those of skill in the art, other designs can be arrived at using the teachings of this disclosure. **FIG.8A** illustrates a perspective view of an intact patella ***800.*** **FIG. 8B** illustrates the patella *800* wherein one surface of the patella ***800*** has been cut for form a smooth surface ***802*** to mate with an implant. **FIG. 8c** illustrates the patella *800* with an implant *810* positioned on the smooth surface ***802.*** The implant ***810*** has a plate structure ***812*** that abuts the smooth surface of the patella ***802*** and a dome ***814*** positioned on the plate ***812*** so that the dome is positioned in situ such that it will match the location of the patellar ridge. The implant ***810*** can be configured such that the edge of the plate is offset 1 mm from the actual edge of the patella, as illustrated. As wll be appreciated by those of skill in the art, the plate ***812*** and dome ***814*** can be formed as a single unit or formed from multiple components. **FIG. 8D** is a side view of the implant ***810*** positioned on the patella ***800.*** As shown, the dome is positioned on the implant such that it is off-center. Optimal positioning of the dome will be determined by the position of the patellar ridge.

Turning now to **FIGS. 8E-F,** the implant *810* is shown superimposed on the unaltered patella ***800*** in order to illustrate that the position of the dome ***814*** of the implant corresponds to the location of the patellar ridge.

**FIGS. 8G-J** illustrate an alternative design for the patellar implant. **FIG. 8G** illustrates the implant ***850*** in its beginning stages as a blank with a flat inferior surface ***852*** having pegs ***854*** extending therefrom for anchoring to the patella. The articular or superior surface ***860*** has a rounded dome ***856,*** and a round plate section ***858*** that can be machined to match the bone cut. The articular surface ***860*** takes on the appearance of a "hat" or somberero, having a dome with a rim. The center of the dome ***856*** is also the center of the bearing surface. The rim ***858*** is cut to conform to the needs of the particular patient. **FIG. 8J** illustrates an implant which has been formed from the blank shown in **FIGS. 8G-I.** **FIG. 8I** shows a plurality of possible cut lines ***862, 862***' for purposes of illustration.

**FIGS. 9A-C** illustrate a lateral view of a knee *1020* having a combination of the implants of implanted thereof. In **FIG. 9A****,** an implant covering the condyle ***900***, is illustrated. Suitable implants can be, for example, those shown in **FIGS. 3-8,** as will be appreciated the portion of the condyle covered anterior to posterior can include the entire weight bearing surface, a portion thereof, or a surface greater than the weight bearing surface. Thus, for example, the implant can be configured to terminate prior to the sulcus terminalis or after the sulcus terminalis (e.g., the groove on the femur that coincides with the area where load bearing on the joint surface stops). As shown in **FIGS. 9A-B,** a patellar implant ***900*** can also be provided. **FIG. 9c** illustrates a knee having a condyle implant ***900,*** a patellar implant ***800*** and an implant for the tibial plateau ***200*.**

**FIGS. 10A-D** provide an alternate view of the coronal plane of a knee joint with one or more implants described above implanted therein. **FIG. 10A** illustrates a knee having a tibial implant ***200*** placed therein. **FIG. 10B** illustrates a knee with a condyle implant ***300*** placed therein. As described above, a plurality of the implants taught herein can be provided within a joint in order to restore joint movement. **FIG. 10c** illustrates a knee joint having two implants therein. First, a tibial implant ***200*** is provided on the tibial plateau and a second implant *300* is provided on the facing condyle. As will be appreciated by those of skill in the art. The implants can be installed such that the implants present each other mating surfaces (as illustrated), or not. For example, where the tibial implant ***200*** is placed in the medial compartment of the knee and the condyle implant ***300*** is placed in the lateral compartment. Other combinations will be appreciated by those of skill in the art. Turning now to **FIG. 10D****,** a tibial implant ***200*** is provided along with a bicompartmental condyle implant ***500***. As discussed above, these implants can be associated with the same compartment of the knee joint, but need not be.

The arthroplasty system can be designed to reflect aspects of the tibial shape, femoral shape and/or patellar shape. Tibial shape and femoral shape can include cartilage, bone or both. Moreover, the shape of the implant can also include portions or all components of other articular structures such as the menisci. The menisci are compressible, in particular during gait or loading. For this reason, the implant can be designed to incorporate aspects of the meniscal shape accounting for compression of the menisci during loading or physical activities. For example, the undersurface ***204*** of the implant ***200*** can be designed to match the shape of the tibial plateau including cartilage or bone or both. The superior surface ***202*** of the implant ***200*** can be a composite of the articular surface of the tibia (in particular in areas that are not covered by menisci) and the meniscus. Thus, the outer aspects of the device can be a reflection of meniscal height. Accounting for compression, this can be, for example, 20%, 40%, 60% or 80% of uncompressed meniscal height.

Similarly the superior surface ***304*** of the implant ***300*** can be designed to match the shape of the femoral condyle including cartilage or bone or both. The inferior surface *302* of the implant ***300*** can be a composite of the surface of the tibial plateau (in particular in areas that are not covered by menisci) and the meniscus. Thus, at least a portion of the outer aspects of the device can be a reflection of meniscal height. Accounting for compression, this can be, for example, 20%, 40%, 60% or 80% of uncompressed meniscal height. These same properties can be applied to the implants shown in **FIGS. 4-8,** as well.

In some examples, the outer aspect of the device reflecting the meniscal shape can be made of another, preferably compressible material. If a compressible material is selected it is preferably designed to substantially match the compressibility and biomechanical behavior of the meniscus. The entire device can be made of such a material or non-metallic materials in general.

The height and shape of the menisci for any joint surface to be repaired can be measured directly on an imaging test. If portions, or all, of the meniscus are torn, the meniscal height and shape can be derived from measurements of a contralateral joint or using measurements of other articular structures that can provide an estimate on meniscal dimensions.

In another embodiment, the superior face of the implants ***300, 400*** or ***500*** can be shaped according to the femur. The shape can preferably be derived from the movement patterns of the femur relative to the tibial plateau thereby accounting for variations in femoral shape and tibiofemoral contact area as the femoral condyle flexes, extends, rotates, translates and glides on the tibia and menisci.

The movement patterns can be measured using any current or future test know in the art such as fluoroscopy, MRI, gait analysis and combinations thereof.

The arthroplasty can have two or more components, one essentially mating with the tibial surface and the other substantially articulating with the femoral component. The two components can have a flat opposing surface. Alternatively, the opposing surface can be curved. The curvature can be a reflection of the tibial shape, the femoral shape including during joint motion, and the meniscal shape and combinations thereof.

Examples of single-component systems include, but are not limited to, a plastic, a polymer, a metal, a metal alloy, crystal free metals, a biologic material or combinations thereof. In certain examples, the surface of the repair system facing the underlying bone can be smooth. In other examples, the surface of the repair system facing the underlying bone can be porous or porous-coated. In another aspect, the surface of the repair system facing the underlying bone is designed with one or more grooves, for example to facilitate the in-growth of the surrounding tissue. The external surface of the device can have a step-like design, which can be advantageous for altering biomechanical stresses. Optionally, flanges can also be added at one or more positions on the device (*e.g*., to prevent the repair system from rotating, to control toggle and/or prevent settling into the marrow cavity). The flanges can be part of a conical or a cylindrical design. A portion or all of the repair system facing the underlying bone can also be flat which can help to control depth of the implant and to prevent toggle.

Non-limiting examples of multiple-component systems include combinations of metal, plastic, metal alloys, crystal free metals, and one or more biological materials. One or more components of the articular surface repair system can be composed of a biologic material (*e.g*. a tissue scaffold with cells such as cartilage cells or stem cells alone or seeded within a substrate such as a bioresorable material or a tissue scaffold, allograft, autograft or combinations thereof) and/or a non-biological material (*e.g*., polyethylene or a chromium alloy such as chromium cobalt).

Thus, the repair system can include one or more areas of a single material or a combination of materials, for example, the articular surface repair system can have a first and a second component. The first component is typically designed to have size, thickness and curvature similar to that of the cartilage tissue lost while the second component is typically designed to have a curvature similar to the subchondral bone. In addition, the first component can have biomechanical properties similar to articular cartilage, including but not limited to similar elasticity and resistance to axial loading or shear forces. The first and the second component can consist of two different metals or metal alloys. One or more components of the system (*e.g*., the second portion) can be composed of a biologic material including, but not limited to bone, or a non-biologic material including, but not limited to hydroxyapatite, tantalum, a chromium alloy, chromium cobalt or other metal alloys.

One or more regions of the articular surface repair system (e.g., the outer margin of the first portion and/or the second portion) can be bioresorbable, for example to allow the interface between the articular surface repair system and the patient's normal cartilage, over time, to be filled in with hyaline or fibrocartilage. Similarly, one or more regions (e.g., the outer margin of the first portion of the articular surface repair system and/or the second portion) can be porous. The degree of porosity can change throughout the porous region, linearly or non-linearly, for where the degree of porosity will typically decrease towards the center of the articular surface repair system. The pores can be designed for in-growth of cartilage cells, cartilage matrix, and connective tissue thereby achieving a smooth interface between the articular surface repair system and the surrounding cartilage.

The repair system (*e.g*., the second component in multiple component systems) can be attached to the patient's bone with use of a cement-like material such as methylmethacrylate, injectable hydroxy- or calcium-apatite materials and the like.

In certain examples, one or more portions of the articular surface repair system can be pliable or liquid or deformable at the time of implantation and can harden later. Hardening can occur, for example, within 1 second to 2 hours (or any time period therebetween), preferably with in 1 second to 30 minutes (or any time period therebetween), more preferably between 1 second and 10 minutes (or any time period therebetween).

One or more components of the articular surface repair system can be adapted to receive injections. For example, the external surface of the articular surface repair system can have one or more openings therein. The openings can be sized to receive screws, tubing, needles or other devices which can be inserted and advanced to the desired depth, for example, through the articular surface repair system into the marrow space. Injectables such as methylmethacrylate and injectable hydroxy- or calcium-apatite materials can then be introduced through the opening (or tubing inserted therethrough) into the marrow space thereby bonding the articular surface repair system with the marrow space. Similarly, screws or pins, or other anchoring mechanisms. can be inserted into the openings and advanced to the underlying subchondral bone and the bone marrow or epiphysis to achieve fixation of the articular surface repair system to the bone. Portions or all components of the screw or pin can be bioresorbable, for example, the distal portion of a screw that protrudes into the marrow space can be bioresorbable. During the initial period after the surgery, the screw can provide the primary fixation of the articular surface repair system. Subsequently, ingrowth of bone into a porous coated area along the undersurface of the articular cartilage repair system can take over as the primary stabilizer of the articular surface repair system against the bone.

The articular surface repair system can be anchored to the patient's bone with use of a pin or screw or other attachment mechanism. The attachment mechanism can be bioresorbable. The screw or pin or attachment mechanism can be inserted and advanced towards the articular surface repair system from a non-cartilage covered portion of the bone or from a non-weight-bearing surface of the joint.

The interface between the articular surface repair system and the surrounding normal cartilage can be at an angle, for example oriented at an angle of 90 degrees relative to the underlying subchondral bone. Suitable angles can be determined in view of the teachings herein, and in certain cases, non-90 degree angles can have advantages with regard to load distribution along the interface between the articular surface repair system and the surrounding normal cartilage.

The interface between the articular surface repair system and the surrounding normal cartilage and/or bone can be covered with a pharmaceutical or bioactive agent, for example a material that stimulates the biological integration of the repair system into the normal cartilage and/or bone. The surface area of the interface can be irregular, for example, to increase exposure of the interface to pharmaceutical or bioactive agents.

### E. PRE-EXISTING REPAIR SYSTEMS

As described herein, repair systems of various sizes, curvatures and thicknesses can be obtained. These repair systems can be catalogued and stored to create a library of systems from which an appropriate system for an individual patient can then be selected. In other words, a defect, or an articular surface, is assessed in a particular subject and a pre-existing repair system having a suitable shape and size is selected from the library for further manipulation (e.g., shaping) and implantation.

### F. MINI-PROSTHESIS

As noted above, the methods and compositions described herein can be used to replace only a portion of the articular surface, for example, an area of diseased cartilage or lost cartilage on the articular surface. In these systems, the articular surface repair system can be designed to replace only the area of diseased or lost cartilage or it can extend beyond the area of diseased or lost cartilage, *e.g*., 3 or 5 mm into normal adjacent cartilage. In certain embodiments, the prosthesis replaces less than about 70% to 80% (or any value therebetween) of the articular surface (*e.g*., any given articular surface such as a single femoral condyle, etc.), preferably, less than about 50% to 70% (or any value therebetween), more preferably, less than about 30% to 50% (or any value therebetween), more preferably less than about 20% to 30% (or any value therebetween), even more preferably less than about 20% of the articular surface.

The prosthesis can include multiple components, for example a component that is implanted into the bone (*e.g*., a metallic device) attached to a component that is shaped to cover the defect of the cartilage overlaying the bone. Additional components, for example intermediate plates, meniscal repair systems and the like can also be included. It is contemplated that each component replaces less than all of the corresponding articular surface. However, each component need not replace the same portion of the articular surface. In other words, the prosthesis can have a bone-implanted component that replaces less than 30% of the bone and a cartilage component that replaces 60% of the cartilage. The prosthesis can include any combination, provided each component replaces less than the entire articular surface.

The articular surface repair system can be formed or selected so that it will achieve a near anatomic fit or match with the surrounding or adjacent cartilage or bone. Typically, the articular surface repair system is formed and/or selected so that its outer margin located at the external surface will be aligned with the surrounding or adjacent cartilage.

Thus, the articular repair system can be designed to replace the weight-bearing portion (or more or less than the weight bearing portion) of an articular surface, for example in a femoral condyle. The weight-bearing surface refers to the contact area between two opposing articular surfaces during activities of normal daily living (*e.g*., normal gait). At least one or more weight-bearing portions can be replaced in this manner, *e.g*., on a femoral condyle and on a tibia.

In other examples, an area of diseased cartilage or cartilage loss can be identified in a weight-bearing area and only a portion of the weight-bearing area, specifically the portion containing the diseased cartilage or area of cartilage loss, can be replaced with an articular surface repair system.

In another example, the articular repair system can be designed or selected to replace substantially all of the articular surface, e.g. a condyle.

Alternatively, in patients with diffuse cartilage loss, the articular repair system can be designed to replace an area slightly larger than the weight-bearing surface.

In certain aspects, the defect to be repaired is located only on one articular surface, typically the most diseased surface. For example, in a patient with severe cartilage loss in the medial femoral condyle but less severe disease in the tibia, the articular surface repair system can only be applied to the medial femoral condyle. Preferably, in any methods described herein, the articular surface repair system is designed to achieve an exact or a near anatomic fit with the adjacent normal cartilage.

In other examples, more than one articular surface can be repaired. The area(s) of repair will be typically limited to areas of diseased cartilage or cartilage loss or areas slightly greater than the area of diseased cartilage or cartilage loss within the weight-bearing surface(s).

In another example, one or more components of the articular surface repair (*e.g*., the surface of the system that is pointing towards the underlying bone or bone marrow) can be porous or porous coated. A variety of different porous metal coatings have been proposed for enhancing fixation of a metallic prosthesis by bone tissue in-growth. Thus, for example, U.S. Pat. No. 3,855,638 to Pilliar issued December 24, 1974, discloses a surgical prosthetic device, which can be used as a bone prosthesis, comprising a composite structure consisting of a solid metallic material substrate and a porous coating of the same solid metallic material adhered to and extending over at least a portion of the surface of the substrate. The porous coating consists of a plurality of small discrete particles of metallic material bonded together at their points of contact with each other to define a plurality of connected interstitial pores in the coating. The size and spacing of the particles, which can be distributed in a plurality of monolayers, can be such that the average interstitial pore size is not more than about 200 microns. Additionally, the pore size distribution can be substantially uniform from the substrate-coating interface to the surface of the coating. In another example, the articular surface repair system can contain one or more polymeric materials that can be loaded with and release therapeutic agents including drugs or other - pharmacological treatments that can be used for drug delivery. The polymeric materials can, for example, be placed inside areas of porous coating. The polymeric materials can be used to release therapeutic drugs, e.g. bone or cartilage growth stimulating drugs. This example can be combined with other examples, wherein portions of the articular surface repair system can be bioresorbable. For example, the first layer of an articular surface repair system or portions of its first layer can be bioresorbable. As the first layer gets increasingly resorbed, local release of a cartilage growth-stimulating drug can facilitate in-growth of cartilage cells and matrix formation.

In any of the methods or compositions described herein, the articular surface repair system can be pre-manufactured with a range of sizes, curvatures and thicknesses. Alternatively, the articular surface repair system can be custom-made for an individual patient.

### IV. MANUFACTURING

### A. SHAPING

In certain instances shaping of the repair material will be required before or after formation (*e.g*., growth to desired thickness), for example where the thickness of the required cartilage material is not uniform (*e.g*., where different sections of the cartilage replacement or regenerating material require different thicknesses).

The replacement material can be shaped by any suitable technique including, but not limited to, casting techniques, mechanical abrasion, laser abrasion or ablation, radiofrequency treatment, cryoablation, variations in exposure time and concentration of nutrients, enzymes or growth factors and any other means suitable for influencing or changing cartilage thickness. See, *e.g*., WO 00/15153 to Mansmann published March 23, 2000; If enzymatic digestion is used, certain sections of the cartilage replacement or regenerating material can be exposed to higher doses of the enzyme or can be exposed longer as a means of achieving different thicknesses and curvatures of the cartilage replacement or regenerating material in different sections of said material.

The material can be shaped manually and/or automatically, for example using a device into which a pre-selected thickness and/or curvature has been input and then programming the device using the input information to achieve the desired shape.

In addition to, or instead of, shaping the cartilage repair material, the site of implantation (*e.g*., bone surface, any cartilage material remaining, etc.) can also be shaped by any suitable technique in order to enhance integration of the repair material.

### B. SIZING

The articular repair system can be formed or selected so that it will achieve a near anatomic fit or match with the surrounding or adjacent cartilage, subchondral bone, menisci and/or other tissue. The shape of the repair system can be based on the analysis of an electronic image (*e.g*. MRI, CT, digital tomosynthesis, optical coherence tomography or the like). If the articular repair system is intended to replace an area of diseased cartilage or lost cartilage, the near anatomic fit can be achieved using a method that provides a virtual reconstruction of the shape of healthy cartilage in an electronic image.

In one example, a near normal cartilage surface at the position of the cartilage defect can be reconstructed by interpolating the healthy cartilage surface across the cartilage defect or area of diseased cartilage. This can, for example, be achieved by describing the healthy cartilage by means of a parametric surface (*e.g*. a B-spline surface), for which the control points are placed such that the parametric surface follows the contour of the healthy cartilage and bridges the cartilage defect or area of diseased cartilage. The continuity properties of the parametric surface will provide a smooth integration of the part that bridges the cartilage defect or area of diseased cartilage with the contour of the surrounding healthy cartilage. The part of the parametric surface over the area of the cartilage defect or area of diseased cartilage can be used to determine the shape or part of the shape of the articular repair system to match with the surrounding cartilage.

In another example, a near normal cartilage surface at the position of the cartilage defect or area of diseased cartilage can be reconstructed using morphological image processing. In a first step, the cartilage can be extracted from the electronic image using manual, semi-automated and/or automated segmentation techniques (*e.g*., manual tracing, region growing, live wire, model-based segmentation), resulting in a binary image. Defects in the cartilage appear as indentations that can be filled with a morphological closing operation performed in 2-D or 3-D with an appropriately selected structuring element. The closing operation is typically defined as a dilation followed by an erosion. A dilation operator sets the current pixel in the output image to 1 if at least one pixel of the structuring element lies inside a region in the source image. An erosion operator sets the current pixel in the output image to 1 if the whole structuring element lies inside a region in the source image. The filling of the cartilage defect or area of diseased cartilage creates a new surface over the area of the cartilage defect or area of diseased cartilage that can be used to determine the shape or part of the shape of the articular repair system to match with the surrounding cartilage or subchondral bone.

As described above, the articular repair system can be formed or selected from a library or database of systems of various sizes, curvatures and thicknesses so that it will achieve a near anatomic fit or match with the surrounding or adjacent cartilage and/or subchondral bone. These systems can be pre-made or made to order for an individual patient. In order to control the fit or match of the articular repair system with the surrounding or adjacent cartilage or subchondral bone or menisci and other tissues preoperatively, a software program can be used that projects the articular repair system over the anatomic position where it will be implanted. Suitable software is commercially available and/or readily modified or designed by a skilled programmer.

In yet another example, the articular surface repair system can be projected over the implantation site using one or more 3-D images. The cartilage and/or subchondral bone and other anatomic structures are extracted from a 3-D electronic image such as an MRI or a CT using manual, semi-automated and/or automated segmentation techniques. A 3-D representation of the cartilage and/or subchondral bone and other anatomic structures as well as the articular repair system is generated, for example using a polygon or NURBS surface or other parametric surface representation. For a description of various parametric surface representations see, for example Foley, J.D. et al., Computer Graphics: Principles and Practice in C; Addison-Wesley, 2nd edition, 1995).

The 3-D representations of the cartilage and/or subchondral bone and other anatomic structures and the articular repair system can be merged into a common coordinate system. The articular repair system can then be placed at the desired implantation site. The representations of the cartilage, subchondral bone, menisci and other anatomic structures and the articular repair system are rendered into a 3-D image, for example application programming interfaces (APIs) OpenGL® (standard library of advanced 3-D graphics functions developed by SGI, Inc.; available as part of the drivers for PC-based video cards, for example from www.nvidia.com for NVIDIA video cards or www.3dlabs.com for 3Dlabs products, or as part of the system software for Unix workstations) or DirectX® (multimedia API for Microsoft Windows® based PC systems; available from www.microsoft.com). The 3-D image can be rendered showing the cartilage, subchondral bone, menisci or other anatomic objects, and the articular repair system from varying angles, *e.g*. by rotating or moving them interactively or non-interactively, in real-time or non-real-time.

The software can be designed so that the articular repair system, including surgical tools and instruments with the best fit relative to the cartilage and/or subchondral bone is automatically selected, for example using some of the techniques described above. Alternatively, the operator can select an articular repair system, including surgical tools and instruments and project it or drag it onto the implantation site using suitable tools and techniques. The operator can move and rotate the articular repair systems in three dimensions relative to the implantation site and can perform a visual inspection of the fit between the articular repair system and the implantation site. The visual inspection can be computer assisted. The procedure can be repeated until a satisfactory fit has been achieved. The procedure can be performed manually by the operator; or it can be computer-assisted in whole or part. For example, the software can select a first trial implant that the operator can test. The operator can evaluate the fit. The software can be designed and used to highlight areas of poor alignment between the implant and the surrounding cartilage or subchondral bone or menisci or other tissues. Based on this information, the software or the operator can then select another implant and test its alignment. One of skill in the art will readily be able to select, modify and/or create suitable computer programs for the purposes described herein.

In another example, the implantation site can be visualized using one or more cross-sectional 2-D images. Typically, a series of 2-D cross-sectional images will be used. The 2-D images can be generated with imaging tests such as CT, MRI, digital tomosynthesis, ultrasound, or optical coherence tomography using methods and tools known to those of skill in the art. The articular repair system can then be superimposed onto one or more of these 2-D images. The 2-D cross-sectional images can be reconstructed in other planes, *e.g*. from sagittal to coronal, etc. Isotropic data sets (*e.g*., data sets where the slice thickness is the same or nearly the same as the in-plane resolution) or near isotropic data sets can also be used. Multiple planes can be displayed simultaneously, for example using a split screen display. The operator can also scroll through the 2-D images in any desired orientation in real time or near real time; the operator can rotate the imaged tissue volume while doing this. The articular repair system can be displayed in cross-section utilizing different display planes, *e.g*. sagittal, coronal or axial, typically matching those of the 2-D images demonstrating the cartilage, subchondral bone, menisci or other tissue. Alternatively, a three-dimensional display can be used for the articular repair system. The 2-D electronic image and the 2-D or 3-D representation of the articular repair system can be merged into a common coordinate system. The articular repair system can then be placed at the desired implantation site. The series of 2-D cross-sections of the anatomic structures, the implantation site and the articular repair system can be displayed interactively (*e.g*. the operator can scroll through a series of slices) or non-interactively (*e.g*. as an animation that moves through the series of slices), in real-time or non-real-time.

### C. RAPID PROTOTYPING

Rapid protyping is a technique for fabricating a three-dimensional object from a computer model of the object. A special printer is used to fabricate the prototype from a plurality of two-dimensional layers. Computer software sections the representations of the object into a plurality of distinct two-dimensional layers and then a three-dimensional printer fabricates a layer of material for each layer sectioned by the software. Together the various fabricated layers form the desired prototype. More information about rapid prototyping techniques is available in US Patent Publication No 2002/0079601A1 to Russell et al., published June 27, 2002. An advantage to using rapid prototyping is that it enables the use of free form fabrication techniques that use toxic or potent compounds safely. These compounds can be safely incorporated in an excipient envelope, which reduces worker exposure.

A powder piston and build bed are provided. Powder includes any material (metal, plastic, etc.) that can be made into a powder or bonded with a liquid. The power is rolled from a feeder source with a spreader onto a surface of a bed. The thickness of the layer is controlled by the computer. The print head then deposits a binder fluid onto the powder layer at a location where it is desired that the powder bind. Powder is again rolled into the build bed and the process is repeated, with the binding fluid deposition being controlled at each layer to correspond to the three-dimensional location of the device formation. For a further discussion of this process see, for example, US Patent Publication No 2003/017365A1 to Monkhouse et al. published September 18, 2003.

The rapid prototyping can use the two dimensional images obtained, as described above in Section I, to determine each of the two-dimensional shapes for each of the layers of the prototyping machine. In this scenario, each two dimensional image slice would correspond to a two dimensional prototype slide. Alternatively, the three-dimensional shape of the defect can be determined, as described above, and then broken down into two dimensional slices for the rapid prototyping process. The advantage of using the three-dimensional model is that the two-dimensional slices used for the rapid prototyping machine can be along the same plane as the two-dimensional images taken or along a different plane altogether.

Rapid prototyping can be combined or used in conjunction with casting techniques. For example, a shell or container with inner dimensions corresponding to an articular repair system can be made using rapid prototyping. Plastic or wax-like materials are typically used for this purpose. The inside of the container can subsequently be coated, for example with a ceramic, for subsequent casting. Using this process, personalized casts can be generated.

Rapid prototyping can be used for producing articular repair systems. Rapid prototyping can be performed at a manufacturing facility. Alternatively, it may be performed in the operating room after an intraoperative measurement has been performed.

### V. SURGICAL TECHNIQUES

Prior to performing surgery on a patient, the surgeon can preoperatively make a determination of the alignment of the knee using, for example, an erect AP x-ray. In performing preoperative assessment any lateral and patella spurs that are present can be identified.

Using standard surgical techniques, the patient is anesthetized and an incision is made in order to provide access to the portion or portions of the knee joint to be repaired. A medial portal can be used initially to enable arthroscopy of the joint. Thereafter, the medial portal can be incorporated into the operative incision and/or standard lateral portals can be used.

Once an appropriate incision has been made, the exposed compartment is inspected for integrity, including the integrity of the ligament structures. If necessary, portions of the meniscus can be removed as well as any spurs or osteophytes that were identified in the AP x-ray or that may be present within the joint. In order to facilitate removal of osteophytes, the surgeon may flex the knee to gain exposure to additional medial and medial-posterior osteophytes. Additionally, osteophytes can be removed from the patella during this process. If necessary, the medial and/or lateral meniscus can also be removed at this point, if desired, along with the rim of the meniscus.

As would be appreciated by those of skill in the art, evaluation of the medial cruciate ligament may be required to facilitate tibial osteophyte removal.

Once the joint surfaces have been prepared, the desired implants can be inserted into the joint.

### A. Tibial Plateau

To insert the device **200** of **FIG. 2** into the medial compartment, perform a mini-incision arthrotomy medial to the patella tendon. Once the incision is made, expose the medial condyle and prepare a medial sleeve to about 1 cm below the joint line using a suitable knife and curved osteotome. After preparing the medial sleeve, place a Z-retractor around the medial tibial plateau and remove anterior portions of the meniscus and the osteophytes along the tibia and femur. At this point, the knee should be flexed to about 60° or more. Remove the Z-retractor and place the implant against the most distal aspect of the femur and over the tibial plateau edge. Push the implant straight back. In some instances, application of valgus stress may ease insertion of the implant.

To insert the device of **FIG. 2** into the lateral compartment, perform a mini-incision arthrotomy lateral to the patella tendon. Once the incision is made, expose the lateral condyle and prepare a lateral sleeve to about 1 cm below the joint line using a suitable knife and curved osteotome. After preparing the lateral sleeve, place a Z-retractor around the lateral tibial plateau and remove anterior portions of the meniscus and the osteophytes along the tibia and femur. Remove the Z-retractor and place the implant against the distal aspect of the femur and over the tibial plateau edge. Hold the implant at a 45° angle and rotate the implant against the lateral condyle using a lateral to medial push toward the lateral spine. In some instances, application of varus stress may ease insertion of the implant.

Once any implant shown in **FIG. 2** is implanted, the device should be positioned within 0 to 2mm of the AP boundaries of the tibial plateau and superimposed over the boundary. Verification of the range of motion should then be performed to confirm that there is minimal translation of the implant. Once positioning is confirmed, closure of the wound is performed using techniques known in the art.

As will be appreciated by those of skill in the art, additional treatment of the surface of the tibial plateau may be desirable depending on the configuration of the implant ***200***. For example, one or more channels or grooves may be formed on the surface of the tibial plateau to accommodate anchoring mechanisms such as the keel ***212*** shown in **FIG. 2K** or the translational movement cross-members ***222, 221*** shown in **FIGS. 2M-N.**

### B. Condylar Repair Systems

To insert the device ***300*** shown in **FIG. 3****,** depending on the condyle to be repaired either an antero-medial or antero-lateral skin incisions is made which begins approximately 1 cm proximal to the superior border of the patella. The incision typically can range from, for example, 6-10 cm along the edge of the patella. As will be appreciated by those of skill in the art, a longer incision may be required under some circumstances.

It may be required to excise excess deep synovium to improve access to the joint. Additionally, all or part of the fat pad may also be excused and to enable inspection of the opposite joint compartment.

Typically, osteophytes are removed from the entire medial and/or lateral edge of the femur and the tibia as well as any osteophytes on the edge of the patella that might be significant.

Although it is possible, typically the devices ***300*** do not require resection of the distal femur prior to implanting the device. However, if desired, bone cuts can be performed to provide a surface for the implant.

At this juncture, the patient's leg is placed in 90° flexion position. I guide can then be placed on the condyle which covers the distal femoral cartilage. The guide enables the surgeon to determine placement of apertures that enable the implant ***300*** to be accurately placed on the condyle. With the guide in place, holes are drilled into the condyle to create apertures from 1-3mm in depth. Once the apertures have been created, the guide is removed and the implant ***300*** is installed on the surface of the condyle. Cement can be used to facilitate adherence of the implant ***300*** to the condyle.

Where more than one condyle is to be repaired, e.g., using two implants ***300*** of **FIG. 3****,** or the implant ***400*** of **FIG. 4****,** or where a condyle and a portion of the patellar surface is to be repaired, e.g., using the implant ***500*** of **FIG. 5****,** the surgical technique described herein would be modified to, for example, provide a greater incision for accessing the joint, provide additional apertures for receiving the pegs of the implant, etc.

### C. Patellar Repair System

To insert the device shown in **FIG. 7****,** it may be appropriate to use the incisions made laterally or medially to the patella tendon and described above with respect to **FIG. 2****.** First the patella is everted laterally and the fat pad and synovium are bent back from around the periphery of the patella. If desired, osteophytes can be removed. Prior to resurfacing the natural patella ***620*,** the knee should be manually taken through several range of motion maneuvers to determine whether subluxation is present. If subluxation is present, then it may be necessary to medialize the implant ***600***. The natural patella can then be cut in a planar, or flat, manner such that a flat surface is presented to the implant. The geometric center of the patella ***620*** is then typically aligned with the geometric center of the implant ***600***. In order to anchor the implant ***600*** to the patella ***620*,** one or more holes or apertures ***612*** can be created in the patellar surface to accept the pegs ***610*** of the implant ***600*.**

### VI. KITS

One ore more of the implants described above can be combined together in a kit such that the surgeon can select one or more implants to be used during surgery.

The foregoing description of embodiments of the present invention has been provided for the purposes of illustration and description. It is not intended to be exhaustive or to limit the invention to the precise forms disclosed. Many modifications and variations will be apparent to the practitioner skilled in the art. The embodiments were chosen and described in order to best explain the principles of the invention and its practical application, thereby enabling others skilled in the art to understand the invention and the various embodiments and with various modifications that are suited to the particular use contemplated. It is intended that the scope of the invention be defined by the following claims equivalents thereof.

Disclosed is an implant suitable for a condyle of a femur having a superior surface and an inferior surface wherein the superior surface opposes at least a portion of the condyle of the femur and the trochlea and the inferior surface opposes at least a portion of a weight bearing portion of a tibial surface and a patella and further wherein at least at least a portion of one of the superior or inferior surfaces has a three-dimensional shape that substantially matches the shape of one of the femur and tibia surfaces.

Further disclosed is the above implant, wherein the superior surface and the inferior surface have a three dimensional shape that substantially matches the shape of at least one of the articular surface that the superior surface of the implant abuts and the inferior surface of the implant abuts.

Also disclosed is the above implant, wherein the implant has a thickness of a cartilage defect in a patient.

Disclosed is that the above implant can have a thickness of 85% of a cartilage defect in a patient.

The implant can have a thickness of between 65%-100% of a cartilage defect of a patient.

The implant can have a thickness of a cartilage defect plus a predefined offset value, wherein said offset value can optionally be selected to adjust for axis malalignment.

Disclosed is the above implant, wherein the implant is constructed of a material comprising metal or metal alloy.

Disclosed is the above implant, wherein the material comprises one or more biologically active materials.

Also disclosed is the above implant, wherein the implant is coated with a biologically active material.

Further disclosed is the above implant, wherein the implant is comprised of a metal or metal alloy and a polymer.

Disclosed is that the above implant can further have a structure for attachment on at least one of the superior surface and the inferior surface selected from the group consisting of: ridges, pegs, pins, cruciate stems, cross-members, teeth and protrusions, optionally wherein the implant further has a plurality of structures for attachment.

Disclosed is also the above implant, wherein the relative orientation of the structures for attachment are selected from the group consisting of: symmetrical, asymmetrical, rows, circles, triangles, and random.

Disclosed is that the above implant can cover a portion of a patellar surface of the femur.

Disclosed is the above implant wherein each of the superior surface and inferior surface have a slope relative to a longitudinal axis through at least a portion of the implant and further wherein the slope of the superior surface relative to the slope of the inferior surface is selected from the group consisting of: positive, negative, and null.

Further disclosed is the above implant, wherein the implant approximates the shape of one of the first and second articular surface.

Likewise disclosed is the above implant, wherein a condyle mating surface of the implant has at least one planar surface for mating with a condyle surface, optionally wherein a femoral condyle surface is prepared for receiving the implant.

Furthermore disclosed is the above implant, wherein the condyle surface is prepared by forming at least one planar surface to mate with the at least one planar surface of the implant.

Disclosed is the above implant, wherein a femoral condyle surface is prepared by removing bone.

Disclosed is that the condyle surface can be prepared by removing cartilage.

Disclosed is also the above implant wherein the condyle surface is prepared by forming at least one planar surface to mate with the at least one planar surface of the implant.

Disclosed is the above implant wherein the implant is selected from a library of implants.

Further disclosed is the above implant wherein the implant is surgical implanted via an incision of 10 cm or less, optionally wherein the implant is surgically implanted via an incision of 6 cm or less.

Also disclosed is the above implant, wherein the range of motion of the joint is restored to between 80-99.9% of normal joint motion, optionally wherein the range of motion of the joint is restored to between 90-99.9% of normal joint motion.

Disclosed is also that the range of motion of the joint can be restored to between 95-99. 9% of normal joint motion. The range of motion of the joint can be restored to between 98-99.9% of normal joint motion.

Further disclosed is the above implant wherein the implant is formed to oppose at least a portion of a second condyle on the femur.

Disclosed is that the above implant can further comprise a rail extending from the superior surface on one of a medial or lateral side.

Also disclosed is that the above implant can further comprise a keel extending from the superior surface, wherein the keel is in a recessed position from the medial and lateral side.

Disclosed is the above implant, wherein a portion of the superior surface sits within a plane, optionally wherein the portion of the superior surface that sits within a plane mates with a planar cut on a surface of the femur.

Also disclosed is the above implant wherein the implant mimics an anatomy of a healthy joint, an anatomy of a joint prior to surgery, or a targeted anatomy of a joint.

Disclosed is that the above implant can be placed within a joint space during a surgery without performing ligament balancing.

Disclosed is the above implant, wherein the implant is a system comprised of a condylar implant and a patella mating implant.

Further disclosed is the above implant, wherein a thickness of the implant at a location is adjusted to account for at least one of valgus deformity, varus deformity and flattening.

Disclosed is that the above implant can further have a beveled edge, a chamfer cut, or a fillet, optionally wherein the fillet is exterior to a posterior portion of a tibial spine.

Disclosed is a kit for repairing a knee comprising one or more implants selected from the following : a condylar implant having a superior surface and an inferior surface wherein the superior surface opposes at least a portion of a condyle of the femur and a trochlea and the inferior surface opposes at least a portion of a weight bearing portion of a tibial surface and a patella and further wherein at least one of the superior or inferior surfaces has a three-dimensional shape that substantially matches the shape of one of the femur and tibia surfaces; a condylar implant having a superior surface and an inferior surface wherein the superior surface opposes at least a portion of a condyle of the femur and the inferior surface opposes at least a portion of a weight bearing portion of a tibial surface and further wherein at least one of the superior or inferior surfaces has a three- dimensional shape that substantially matches the shape of one of the femur and tibia surfaces ; a patellar implant having a first surface that engages the femur mating surface of the patella and a second surface that engages the trochlea ; and an implant suitable for the tibial plateau having a superior surface and in inferior surface wherein the superior surface opposes at least a portion of a femur and the inferior portion opposes at least a portion of the tibial surface and further wherein at least one of the superior or inferior surfaces has a three- dimensional shape that substantially matches the shape of one of the femur and tibial surfaces.

Disclosed is also a prosthetic device for a knee joint comprising: a femoral condyle component having a superior surface and an inferior surface and a top portion and a bottom portion with a curved lateral edge extending therebetween; and a trochlear groove component along the top portion of the device, wherein the bottom portion of the femoral condyle component terminates prior to a sulcus terminals on the joint surface.

Disclosed is the above prosthetic device, wherein the device has a thickness determined by a cartilage defect in a patient.

Disclosed is the above prosthetic device, wherein the device has a thickness of about 85% of a cartilage defect in a patient.

Disclosed is the above prosthetic device, wherein the device has a thickness of between 65% and 100% of a cartilage defect in a patient.

Also disclosed is the above prosthetic device, wherein the device has a thickness of the cartilage defect in a patient plus a predetermined offset value.

Further disclosed is the above prosthetic device, wherein the device is constructed of a material comprising metal or metal alloy.

Disclosed is that the above prosthetic device can have a structure for attachment on at least one surface, optionally wherein the structure for attachment is selected from ridges, pegs, pins, cross-members, cruciate stems, teeth and protrusions.

Disclosed is the above prosthetic device, wherein the implant further comprises a rail extending from the superior surface on one of a medial or lateral side.

Likewise disclosed is the above prosthetic device, wherein the implant further comprises a keel extending from the superior surface, wherein the keel is in a recessed position from the medial and lateral side.

Furthermore disclosed is the above prosthetic device, wherein a portion of the superior surface sits within a plane, optionally wherein the portion of the superior surface that sits within a plane mates with a planar cut on a surface of the femur, or wherein the portion of the superior surface that sits within a plane substantially mates with a planar cut on a surface of the femur.

Also disclosed is a prosthetic device for a knee joint comprising: a femoral condyle component having a top portion and a bottom portion with a curved lateral edge therebetween ; and a trochlear groove component along the top portion of the device, wherein the bottom portion of the femoral condyle component terminates at a sulcus terminalis on the knee joint surface.

Disclosed is the above prosthetic device, wherein the device has a thickness determined by a cartilage defect in a patient.

Also disclosed is the above prosthetic device, wherein the device has a thickness of about 85% of a cartilage defect in a patient.

Further disclosed is that the device has a thickness of between 65% and 100% of a cartilage defect in a patient.

Disclosed is the above prosthetic device can have a thickness of the cartilage defect in a patient plus a predetermined offset value.

Disclosed is that the above prosthetic device can be constructed of a material comprising metal or metal alloy.

Also disclosed is that the above prosthetic device can further have a structure for attachment on at least one surface.

Disclosed is the above prosthetic device, wherein the structure for attachment is selected from ridges, pegs, pins, cross-members, cruciate stems, teeth and protrusions.

Likewise disclosed is the above prosthetic device, wherein the implant further comprises a rail extending from the superior surface on one of a medial or lateral side.

Further disclosed is the above prosthetic device, wherein the implant further comprises a keel extending from the superior surface, wherein the keel is in a recessed position from the medial and lateral side.

Disclosed is the above prosthetic device, wherein a portion of the superior surface sits within a plane, optionally wherein the portion of the superior surface that sits within a plane mates with a planar cut on a surface of the femur, or wherein the portion of the superior surface that sits within a plane substantially mates with a planar cut on a surface of the femur.

Also disclosed is the above prosthetic device, wherein a femoral condyle surface is prepared for receiving the implant, optionally wherein the femoral condyle surface is prepared by forming at least one planar surface to mate with the at least one planar surface of the implant.

Disclosed is the above implant, wherein a femoral condyle surface is prepared by removing bone, optionally wherein the condyle surface is prepared by removing cartilage, or wherein the condyle surface is prepared by forming at least one planar surface to mate with the at least one planar surface of the implant.

Disclosed is the above implant, wherein the implant mimics an anatomy of a healthy joint, of a joint prior to surgery, or wherein the implant mimics a targeted anatomy of a joint.

Further disclosed is the above implant, wherein the implant is placed within a joint space during a surgery without performing ligament balancing.

Disclosed is that the implant can be a system comprised of a condylar implant and a patellar mating implant.

Disclosed is the above implant, wherein athickness of the implant at a location is adjusted to account for at least one of valgus deformity, varus deformity and flattening.

Disclosed is that the above implant further can have a beveled edge, a chamfer cut, or a fillet, optionally wherein the fillet is exterior to a posterior portion of a tibial spine.

Disclosed is an implant suitable for a distal femur in a knee joint having a superior surface and an inferior surface wherein the superior surface is configured to communicate with the femoral surface of a tibiofemoral articulation surface and the inferior surface is configured to communicate with the tibial surface of the tibiofemoral articulation surface.

Likewise disclosed is the above implant, wherein prior to an implantation step, a bone cut is performed on the surface of the femur.

Disclosed is the above implant, wherein the bone cut is posterior.

Also disclosed is the above implant, wherein the implant has a high posterior extension along the femoral condyle, optionally wherein the knee joint has a high degree of knee flexion.

Further disclosed is the above implant, wherein at least a portion of one of the superior surface or inferior surface has a three-dimensional shape that substantially matches the shape of the joint surface with which it mates.

Disclosed is the above implant, wherein the portion of one of the superior surface or inferior surface is greater than 2 cm in length.

Also disclosed is the above implant, wherein the implant further comprises a rail extending from the superior surface on one of a medial or lateral side.

Further disclosed is the above implant, wherein the implant further comprises a keel extending from the superior surface, wherein the keel is in a recessed position from the medial and lateral side.

Further disclosed is the above implant, wherein a portion of the superior surface sits within a plane, optionally wherein the portion of the superior surface that sits within a plane mates with a planar cut on a surface of the femur.

Disclosed is the above implant, wherein the portion of the superior surface that sits within a plane substantially mates with a planar cut on a surface of the femur.

Disclosed is that the above implant further can have an attachment structure, wherein the structure for attachment is selected from ridges, pegs, pins, cross-members, cruciate stems, teeth and protrusions.

Disclosed is an implant system comprising: a femoral component, wherein the femoral component replaces a femoral surface of the patellofemoral articulation surface and a tibiofemoral articulation surface; and a tibial component, wherein the tibial component replaces a tibial surface of the tibiofemoral articulation surface.

Also disclosed is the above implant system, wherein the femoral component has a thickness determined by a cartilage defect in a patient.

Also disclosed is the above implant system, wherein the tibial component has a thickness determined by a cartilage defect in a patient.

Furthermore disclosed is the above implant system, wherein the femoral component has a thickness of about 85% of a cartilage defect in a patient.

Likewise disclosed is the above implant system, wherein the femoral component has a thickness of between 65% and 100% of a cartilage defect in a patient.

Also disclosed is the above implant system, wherein the femoral component has a thickness of the cartilage defect in a patient plus a predetermined offset value.

Disclosed is the above implant system, wherein the femoral component is constructed of a material comprising metal or metal alloy.

Also disclosed is that the above implant system further can have a structure for attachment on at least one surface, optionally wherein the structure for attachment is selected from ridges, pegs, pins, cross-members, cruciate stems, teeth and protrusions.

Disclosed is the above implant system, wherein the superior surface of the femoral component has a substantially flat section designed to mate with a bone cut on a femoral condyle.

Further disclosed is the above implant system, wherein one or more components are sized using x-ray sizing.

Disclosed is also the above implant system, wherein one or more components are manufactured using standard casting techniques.

Also disclosed is the above implant system, wherein the implant further comprises a rail extending from the superior surface on one of a medial or lateral side.

Likewise disclosed is the above implant system, wherein the implant further comprises a keel extending from the superior surface, wherein the keel is in a recessed position from the medial and lateral side.

Disclosed is the above implant system, wherein a portion of the superior surface sits within a plane, optionally wherein the portion of the superior surface that sits within a plane mates with a planar cut on a surface of the femur.

Further disclosed is the above implant system, wherein the portion of the superior surface that sits within a plane substantially mates with a planar cut on a surface of the femur.

Also disclosed is the above implant system, wherein the tibial component has a dome positioned on a plate, optionally wherein the dome is positioned on the plate such that an apex of the dome is off-center a center point of the plate. An apex of the dome can be positioned to correspond to a patella ridge.

Disclosed is the above implant system, wherein a femoral condyle surface is prepared for receiving the implant, optionally wherein the femoral condyle surface is prepared by forming at least one planar surface to mate with the at least one planar surface of the implant, or wherein the femoral condyle surface is prepared by removing cartilage, or wherein the femoral condyle surface is prepared by forming at least one planar surface to mate with the at least one planar surface of the implant.

Also disclosed is the above implant system, wherein the implant mimics an anatomy of a healthy joint, an anatomy of a joint prior to surgery, or wherein the implant mimics a targeted anatomy of a joint.

Further disclosed is the above implant system, wherein the implant is placed within a joint space during a surgery without performing ligament balancing.

Also disclosed is that the above implant system, canbe a system comprised of a condylar implant and a patella mating implant.

Disclosed is the above implant system, wherein a thickness of the implant at a location is adjusted to account for at least one of valgus deformity, varus deformity and flattening.

Also disclosed is the above implant can further have a beveled edge, a chamfer cut, or a fillet, optionally wherein the fillet is exterior to a posterior portion of a tibial spine.

Disclosed is an implant system comprising: a femoral component, wherein the femoral component replaces a femoral surface of the patellofemoral articulation surface and a tibiofemoral articulation surface ; a tibial component, wherein the tibial component replaces the tibial surface of the tibiofemoral articulation surface; and a patella component designed to replace a patella surface of the patellofemoral articulation surface.

Disclosed is the above implant system, wherein the femoral component has a thickness determined by a cartilage defect in a patient.

Disclosed is the above implant system, wherein the tibial component has a thickness determined by a cartilage defect in a patient.

Also disclosed is the above implant system, wherein the femoral component has a thickness of about 85% of a cartilage defect in a patient.

Also disclosed is the above implant system, wherein the femoral component has a thickness of between 65% and 100% of a cartilage defect in a patient.

The femoral component of the above implant system can have a thickness of the cartilage defect in a patient plus a predetermined offset value.

The femoral component of the above implant system can be constructed of a material comprising metal or metal alloy.

Disclosed is that the above implant system can further have a structure for attachment on at least one surface, optionally wherein the structure for attachment is selected from ridges, pegs, pins, cross-members, roll-bars, cruciate stems, teeth and protrusions.

Likewise disclosed is the above implant system, wherein the superior surface of the femoral component has a substantially flat section designed to mate with a bone cut on the femoral condyle.

Also disclosed is the above implant system, wherein one or more components are sized using x-ray sizing.

Disclosed is the above implant system, wherein one or more components are manufactured using standard casting techniques.

Also disclosed is the above implant system, wherein the implant further comprises a rail extending from the superior surface on one of a medial or lateral side, or wherein the implant further comprises a keel extending from the superior surface, wherein the keel is in a recessed position from the medial and lateral side.

Furthermore disclosed is the above implant system, wherein a portion of the superior surface sits within a plane, optionally wherein the portion of the superior surface that sits within a plane mates with a planar cut on a surface of the femur.

Also disclosed is the above implant system, wherein the portion of the superior surface that sits within a plane substantially mates with a planar cut on a surface of the femur.

Disclosed is the above implant system, wherein the tibial component has a dome positioned on a plate, optionally wherein the dome is positioned on the plate such that an apex of the dome is off-center a center point of the plate, or wherein an apex of the dome is positioned to correspond to a patella ridge.

Also disclosed is the above implant system, wherein a femoral condyle surface is prepared for receiving the implant, optionally wherein the femoral condyle surface is prepared by forming at least one planar surface to mate with the at least one planar surface of the implant, or wherein the femoral condyle surface is prepared by removing cartilage, or wherein the femoral condyle surface is prepared by forming at least one planar surface to mate with the at least one planar surface of the implant.

Further disclosed is the above implant, wherein the implant mimics an anatomy of a healthy joint, or of a joint prior to surgery, or wherein the implant mimics a targeted anatomy of a joint.

Also disclosed is the above implant, wherein the implant is placed within a joint space during a surgery without performing ligament balancing.

Further disclosed is the above implant, wherein the implant is a system comprised of a condylar implant and a patella mating implant.

Further disclosed is the above implant, wherein a thickness of the implant at a location is adjusted to account for at least one of valgus deformity, varus deformity and flattening.

Also disclosed is the above implant, wherein further having a beveled edge, a chamfer cut, or a fillet, optionally wherein the fillet is exterior to a posterior portion of a tibial spine.

Disclosed is a prosthetic device for a knee joint comprising: a femoral condyle component having a top portion and a bottom portion with a curved lateral edge therebetween; and a trochlear groove component along the top portion of the device, wherein the bottom portion of the femoral condyle component terminates before a sulcus terminals on the knee joint surface.

Also disclosed is a prosthetic device for a knee joint comprising: a femoral condyle component having a top portion and a bottom portion with a curved lateral edge therebetween ; and a trochlear groove component along the top portion of the device, wherein the bottom portion of the femoral condyle component terminates near a sulcus terminalis on the knee joint surface.

Likewise disclosed is prosthetic device for a knee joint comprising: a femoral condyle component having a top portion and a bottom portion with a curved lateral edge therebetween ; and a trochlear groove component along the top portion of the device, wherein the bottom portion of the femoral condyle component terminates beyond a sulcus terminalis on the knee joint surface.

Morover disclosed is an implant system comprising: a femoral component, wherein the femoral component replaces a femoral surface of the patellofemoral articulation surface and a tibiofemoral articulation surface; and a tibia) component, wherein the tibial component replaces the tibial surface of the tibiofemoral articulation surface, wherein at least one of the tibial component and femoral component is asymmetric.

Also disclosed is implant system comprising: a femoral component, wherein the femoral component replaces a femoral surface of the patellofemoral articulation surface and a tibiofemoral articulation surface ; a tibial component, wherein the tibial component replaces the tibial surface of the tibiofemoral articulation surface; and a patella component designed to replace a patellar surface of the patellofemoral articulation surface wherein at least one of the tibial, femoral and patella component is asymetrical.

Disclosed is also a prosthetic device for a knee joint comprising: a femoral condyle component having a superior surface and an inferior surface and a top portion and a bottom portion with a curved surface extending therebetween; and a trochlear groove component along the top portion of the device, wherein the bottom portion of the femoral condyle component terminates after a sulcus terminalis.

Disclosed is a tibial implant having a first surface having a substantially planar surface and a second surface with a partially planar surface wherein the second surface with a partially planar surface further comprises a dome structure.

Disclosed is the above tibial implant, wherein the dome has an apex positioned to correspond to a patella ridge.

Also disclosed is the above tibial implant, wherein the dome is positioned on the second surface such that the apex of the dome is off- center to a center of the implant.

Furthermore disclosed is a tibial implant having a first surface having a substantially planar surface and a second surface with a partially planar surface wherein the second surface with a partially planar surface further comprises a dome structure located on the partially planar surface such that the planar surface forms a lip around the dome.

Disclosed is the above tibial implant, wherein the lip around the dome is symmetrical around the circumference of the dome, or wherein the lip around the dome is asymmetrical around the circumference of the dome.

Further disclosed is the above tibial implant, wherein the implant forms a blank.

Also disclosed is the above tibial implant, wherein an edge of the planar surface is modified in a patient-specific manner.

## Claims

1. An implant (400) for performing a total knee arthroplasty on a target knee joint (1020) of a patient, the knee joint comprising a lateral condyle and a medial condyle, each of the condyles having a known shape, the implant comprising:
a lateral condyle component (410);
a medial condyle component (420); and
a bridge (430) connecting the lateral condyle component (410) to the medial condyle component (420);
wherein the implant (400) further comprises a superior surface (402) and an inferior surface (404), wherein the superior surface (402) is shaped to correspond to a shape of the condyles such that it corresponds to the actual shape of one or both of the condyles or to a shape of one or both of the condyles after resurfacing of the joint, **characterized in that** the implant is patient-specific, wherein the inferior surface (404) is configured to conform to a projected shape of the condyles (1002, 1004) of the target knee joint (1020) of the patient

2. The implant (400) of claim 1, wherein the inferior surface (404) is configured to conform to the projected shape of a non-damaged portion of the femoral condyles (1002, 1004) of the target knee joint (1020) of the patient.

3. The implant (400) of claim 1, wherein the lateral condyle component (410) is configured to cover the lateral femoral condyle (1002) of the target knee joint (1020) of the patient.

4. The implant (400) of claim 1, wherein the medial condyle component (420) is configured to cover the medial femoral condyle (1004) of the target knee joint (1020) of the patient.

5. The implant (400) of claim 1, wherein the bridge (430) is configured to cover at least a portion of the patellar surface of the femur (1012) of the target knee joint (1020) of the patient.

6. The implant (400) of claim 1, further comprising an implant suitable for implantation on a tibial plateau of a patient.

7. The implant (400) of claim 1, wherein the superior surface of the implant (400) is shaped to correspond to the shape of one or both of the medial (1004) and lateral (1002) femoral condyles of the patient after resurfacing of the joint (1020) of the patient, wherein the resurfacing includes one or more bone cuts of the joint.

8. The implant (400) of claim 1, wherein a first portion of the superior surface (402) contacting a first condyle (1002, 1004) is configured to mate with the existing surface of the condyle (1002, 1004), and a second portion of a superior surface (402) contacting a second condyle (1002, 1004) has one or more flat surfaces to mate with a modified condyle surface of the joint (1020) of the patient.

9. An implant (500) suitable for repairing a condyle (1002, 1004) of a femur (1000) of a target knee joint (1020) of a patient, the knee joint comprising a condyle and at least a portion of a patellar surface having a known shape, the implant comprising:
a superior surface (502) configured to mate with the condyle (1002, 1004) and the portion of a patellar surface of the femur (1012) of the target knee joint (1020) of the patient; and
an inferior surface (504); wherein the superior surface is shaped to correspond to a shape of the condyle that it mates with and the portion of the patellar surface that it covers such that it corresponds to the actual shape of the condyle and the portion of the patellar surface or a shape of the condyle and the portion of the patellar surface after resurfacing of the joint, **characterized in that** the implant is patient-specific, wherein the inferior surface (504) is configured to conform to a projected shape of the condyle (1002, 1004) of the target knee joint (1020) of the patient.

10. The implant (500) of claim 9, wherein the superior surface (502) includes one or more flat sections configured to mate with one or more bone cuts on a femoral condyle (1002, 1004) of the target knee joint (1020) of the patient.

11. The implant (500) of claim 9, wherein the superior surface (502) is shaped to correspond to the shape of existing cartilage or subchondral bone of the femoral condyle (1002, 1004) of the target knee joint (1020) of the patient.

12. The implant (500) of claim 9, wherein the superior surface (502) includes at least one angled surface (503) configured to conform to a corresponding bone cut on the condyle (1002, 1004).

## Patentansprüche

1. Ein Implantat (400) zur Durchführung einer Gesamt-Knieendoprothetik an einem Kniegelenk (1020) eines Patienten, wobei das Kniegelenk einen lateralen Kondylus und einen medialen Kondylus umfasst, wobei jeder der Kondylen eine bekannte Form hat, das Implantat umfassend:
eine laterale Kondylen-Komponente (410);
eine mediale Kondylen Komponente (420); und
eine Brücke (430), die die laterale Kondylen-Komponente (410) mit der medialen Kondylen Komponente (420) verbindet;
worin das Implantat (400) ferner eine obere Oberfläche (402) und eine untere Oberfläche (404) umfasst, worin die obere Oberfläche (402) so geformt ist, dass sie mit einer Form der Kondylen korrespondiert, so dass sie mit der eigentlichen Form von einer oder beiden Kondylen oder mit einer Form von einer oder beiden Kondylen nach dem Gelenk-Resurfacing korrespondiert, wobei es dadurch charakterisiert ist, dass es Pateinten-spezifisch ist, worin die untere Oberfläche (404) so konfiguriert ist, dass sie einer projizierten Form der Kondylen (1002, 1004) des Ziel-Kniegelenks (1020) des Pateinten entspricht.

2. Das Implantat (400) nach Anspruch 1, worin die untere Oberfläche (404) so konfiguriert ist, dass sie der projizierten Form eines nicht-beschädigten Teils der femoralen Kondylen (1002, 1004) des Ziel-Kniegelenks (1020) des Patienten entspricht.

3. Das Implantat (400) nach Anspruch 1, worin die laterale Kondylen-Komponente (410) so konfiguriert ist, dass sie den lateralen femoralen Kondylus (1002) des Ziel-Kniegelenks (1020) des Patienten bedeckt.

4. Das Implantat (400) nach Anspruch 1, worin die mediale Kondylen-Komponente (420) so konfiguriert ist, dass sie den medialen femoralen Kondylus (1004) des Ziel-Kniegelenks (1020) des Patienten bedeckt.

5. Das Implantat (400) nach Anspruch 1, worin die Brücke (430) so konfiguriert ist, dass sie mindestens einen Teil der Patella-Oberfläche des Femurs (1012) des Ziel-Kniegelenks (1020) des Patienten bedeckt.

6. Das Implantat (400) nach Anspruch 1, ferner umfassend ein Implantat, das geeignet ist für die Implantation auf einem Tibia-Plateau eines Patienten.

7. Das Implantat (400) nach Anspruch 1, worin die obere Oberfläche des Implantats (400) so geformt ist, dass sie mit der Form einer oder beider der medialen (1004) und lateralen (1002) femoralen Kondylen des Patienten nach dem "Resurfacing" des Gelenks (1020) des Patienten übereinstimmt, worin das "Resurfacing" einen oder mehrere Knochenschnitte des Gelenks einschließt.

8. Das Implantat (400) nach Anspruch 1, worin ein erster Teil der oberen Oberfläche (402), der einen ersten Kondylus (1002, 1004) berührt, so konfiguriert ist, das er sich mit der existierenden Oberfläche des Kondylus (1002, 1004) verbindet und ein zweiter Teil der oberen Oberfläche (402) der einen zweiten Kondylus (1002, 1004) berührt, ein oder mehrere flache Oberflächen hat, um sich mit der modifizierten Kondylen-Oberfläche des Gelenks (1020) des Pateinten zu verbinden.

9. Ein Implantat (500) geeignet zum Reparieren eines Kondylus (1002, 1004) eines Femurs (1000) eines Ziel-Kniegelenks (1020) eines Patienten, wobei das Kniegelenk einen Kondylus und mindestens einen Teil einer Patella-Oberfläche, die eine bekannte Form hat, umfasst und das Implantat
eine obere Oberfläche (502), die so konfiguriert ist, dass sie sich mit dem Kondylus (1002, 1004) und dem Teil der Patella-Oberfläche des Femurs (1012) des Ziel-Kniegelenks (1020) des Patienten verbindet; und
eine untere Oberfläche (504), worin die obere Oberfläche so geformt ist, dass sie einer Form des Kondylus, mit der sie sich verbindet, und dem Teil der Patella-Oberfläche, die sie bedeckt, entspricht, so dass sie der eigentlichen Form des Kondylus und des Teils der Patella-Oberfläche nach "Resurfacing" des Gelenks entspricht, umfasst,
dadurch charakterisiert ist, dass das Implantat Patienten-spezifisch ist, worin die untere Oberfläche (504) so konfiguriert ist, dass sie einer projizierten Form des Kondylus (1002, 1004) des Ziel-Kniegelenks (1020) des Patienten entspricht.

10. Das Implantat (500) von Anspruch 9, worin die obere Oberfläche (502) ein oder mehrere flache Bereiche einschließt, die so konfiguriert sind, dass sie sich mit einem oder mehreren Knochenschnitten auf einem femoralen Kondylus (1002, 1004) des Ziel-Kniegelenks (1020) des Patienten verbinden.

11. Das Implantat (500) von Anspruch 9, worin die obere Oberfläche (502) so geformt ist, dass sie der Form des existierenden Knorpels oder subchondralen Knochens des femoralen Kondylus (1002, 1004) des Ziel-Kniegelenks (1020) des Pateinten entspricht.

12. Das Implantat (500) von Anspruch 9, worin die obere Oberfläche (502) mindestens eine abgewinkelte Oberfläche (503) einschließt, die so konfiguriert ist, dass sie einem korrespondierenden Knochenschnitt auf dem Kondylus (1002, 1004) entspricht.

## Revendications

1. Implant (400) pour la réalisation d'une arthroplastie totale du genou sur une articulation de genou cible (1020) d'un patient, l'articulation de genou comprenant un condyle latéral et un condyle médial, chacun des condyles ayant une forme connue, l'implant comprenant :
un composant condyle latéral (410) ;
un composant condyle médial (420) ; et
un pont (430) raccordant le composant condyle latéral (410) au composant condyle médial (420) ;
dans lequel l'implant (400) comprend en outre une surface supérieure (402) et une surface inférieure (404), dans lequel la surface supérieure (402) est façonnée de manière à correspondre à une forme des condyles de telle sorte qu'elle correspond à la forme réelle de l'un ou des deux condyles ou à une forme de l'un ou des deux condyles après resurfaçage de l'articulation, **caractérisé en ce que** l'implant est spécifique du patient, dans lequel la surface inférieure (404) est configurée pour se conformer à une forme projetée des condyles (1002, 1004) de l'articulation de genou cible (1020) du patient.

2. Implant (400) selon la revendication 1, dans lequel la surface inférieure (404) est configurée pour se conformer à la forme projetée d'une partie non endommagée des condyles fémoraux (1002, 1004) de l'articulation de genou cible (1020) du patient.

3. Implant (400) selon la revendication 1, dans lequel le composant condyle latéral (410) est configuré pour couvrir le condyle fémoral latéral (1002) de l'articulation de genou cible (1020) du patient.

4. Implant (400) selon la revendication 1, dans lequel le composant condyle médial (420) est configuré pour couvrir le condyle fémoral médial (1004) de l'articulation de genou cible (1020) du patient.

5. Implant (400) selon la revendication 1, dans lequel le pont (430) est configuré pour couvrir au moins une partie de la surface patellaire du fémur (1012) de l'articulation de genou cible (1020) du patient.

6. Implant (400) selon la revendication 1, comprenant en outre un implant approprié pour l'implantation sur un plateau tibial d'un patient.

7. Implant (400) selon la revendication 1, dans lequel la surface supérieure de l'implant (400) est façonnée pour correspondre à la forme de l'un ou des deux des condyles fémoraux médial (1004) et latéral (1002) du patient après le resurfaçage de l'articulation (1020) du patient, dans lequel le resurfaçage comprend une ou plusieurs découpes d'os de l'articulation.

8. Implant (400) selon la revendication 1, dans lequel une première partie de la surface supérieure (402) en contact avec un premier condyle (1002, 1004) est configurée pour s'accoupler à la surface existante du condyle (1002, 1004), et une seconde partie d'une surface supérieure (402) en contact avec un second condyle (1002, 1004) a une ou plusieurs surfaces plates pour s'accoupler à une surface de condyle modifiée de l'articulation (1020) du patient.

9. Implant (500) approprié pour réparer un condyle (1002, 1004) d'un fémur (1000) d'une articulation de genou cible (1020) d'un patient, l'articulation de genou comprenant un condyle et au moins une partie d'une surface patellaire ayant une forme connue, l'implant comprenant :
une surface supérieure (502) configurée pour s'accoupler au condyle (1002, 1004) et à la partie d'une surface patellaire du fémur (1012) de l'articulation de genou cible (1020) du patient ; et
une surface inférieure (504) ; dans lequel la surface supérieure est façonnée pour correspondre à une forme du condyle avec lequel elle s'accouple et à la partie de la surface patellaire qu'elle couvre de telle sorte qu'elle correspond à la forme réelle du condyle et de la partie de la surface patellaire ou à une forme du condyle et de la partie de la surface patellaire après le resurfaçage de l'articulation,
**caractérisé en ce que** l'implant est spécifique du patient, dans lequel la surface inférieure (504) est configurée pour se conformer à une forme projetée du condyle (1002, 1004) de l'articulation de genou cible (1020) du patient.

10. Implant (500) selon la revendication 9, dans lequel la surface supérieure (502) comprend une ou plusieurs sections plates configurées pour s'accoupler à une ou plusieurs découpes d'os sur un condyle fémoral (1002, 1004) de l'articulation de genou cible (1020) du patient.

11. Implant (500) selon la revendication 9, dans lequel la surface supérieure (502) est façonnée pour correspondre à la forme du cartilage ou de l'os sous-chondral existant du condyle fémoral (1002, 1004) de l'articulation de genou cible (1020) du patient.

12. Implant (500) selon la revendication 9, dans lequel la surface supérieure (502) comprend au moins une surface angulaire (503) configurée pour se conformer à une découpe d'os correspondante sur le condyle (1002, 1004).
